# EUROPEAN PATENT SPECIFICATION

(11) **EP 1 717 312 B1**
(45) Date of publication and mention of the grant of the patent: **22.09.2010**
(21) Application number: 05710340.0
(22) Date of filing: 17.02.2005
(51) Int. Cl.: C12N 15/09, C12M 1/00, C12M 1/34, C12Q 1/68, G01N 33/53, G01N 33/566, G01N 37/00

(54) **DNA ARRAY FOR ANALYZING DNA METHYLATION, METHOD OF CONSTRUCTING THE SAME AND MEHTOD OF ANALYZING DNA METHYLAION**
DNA-ARRAY ZUR ANALYSE DER DNA-METHYLIERUNG, KONSTRUKTIONSVERFAHREN DAFÜR SOWIE VERFAHREN ZUR ANALYSE DER DNA-METHYLIERUNG
PUCE A ADN POUR L'ANALYSE DE LA METHYLATION DE L'ADN ET SON PROCEDE DE FABRICATION, ET PROCEDE D'ANALYSE DE LA METHYLATION DE L'ADN

(30) Priority: 20.02.2004 JP 2004044759
(43) Date of publication of application: 02.11.2006
(73) Proprietor: Japan Science and Technology Agency, Kawaguchi-shi, Saitama 332-0012 (JP); Tokyo Metropolitan Geriatric Hospital and Institute of Gerontology, Tokyo 173-0015 (JP)
(72) Inventor: YAMAKAWA, Naomi, Itabashi-ku Tokyo 173-0015 (JP)
(74) Representative: Cohausz & Florack
(86) International application number: PCT/JP2005/002490
(87) International publication number: WO 2005/080565

(56) References cited:
- WO-A-02/00842
- WO-A1-2004/104582
- JP-A- 3 022 981
- JP-A- 2003 038 183
- FISHER OHAD ET AL: "Characterization of cytosine methylated regions and 5-cytosine DNA methyltransferase (Ehmeth) in the protozoan parasite Entamoeba histolytica." NUCLEIC ACIDS RESEARCH, vol. 32, no. 1, January 2004 (2004-01), pages 287-297, XP002451867 ISSN: 0305-1048
- LUO SONG ET AL: "Strand-biased DNA methylation associated with centromeric regions in Arabidopsis." PROCEEDINGS OF THE NATIONAL ACADEMY OF SCIENCES OF THE UNITED STATES OF AMERICA, vol. 100, no. 19, 16 September 2003 (2003-09-16), pages 11133-11138, XP002451868 ISSN: 0027-8424
- KUMAR R R ET AL: "IMMUNOAFFINITY CHROMATOGRAPHY TO ISOLATE METHYLATED DNA USING IMMOBILIZED ANTI-5-METHYLCYTOSINE ANTIBODY" BIOTECHNOLOGY TECHNIQUES, vol. 5, no. 6, 1991, pages 469-470, XP008083955 ISSN: 0951-208X
- SUZUKI Y. ET AL: 'Keiko Men'eki Senshoku ni yoru Senshokutai DNA Ko-Methyl ka Ryoiki no Kenshutsu.' THE MOLECULAR BIOLOGY SOCIETY OF JAPAN. vol. 25, 2002, page 717, XP008093098

## Description

### TECHNICAL FIELD

The present invention relates to a DNA array for analyzing a DNA modification (particularly DNA methylation), a method of producing the DNA array, and a method of analyzing a DNA modification (particularly DNA methylation). Further, the present invention relates to a method of purifying or obtaining a DNA fragment.

### BACKGROUND ART

Genetic information of organisms is integrated into DNAs. In higher animals, nucleotide sequences (i.e., genetic information) are the same among almost all types of cells which form tissues. However, a phenotype of each cell is determined by regulating an expression of each gene, and methylation in genomic DNA plays an important role. The DNAs in an ovum and a sperm are reset immediately after fertilization, and reprogramming is carried out. In an individual, after the generation, the state of methylation in DNAs varies slightly with cell divisions, and a relationship between methylation and senescence is noted. When there is an unfavorable change in the methylation of the genome together with aging, gene expressions unfavorable for an individual, such as a suppression of a gene to be expressed or an expression of a gene to be inhibited, sometimes occur. Such an abnormal methylation of DNAs is also observed in cancer cells. In particular, an inactivation of tumor suppressor genes caused by hypermethylation in CpG islands is frequently observed.

As above, methylation in DNA chains is an important index for various diseases including cancers. Further, since methylation is involved in the regulation of gene expression, methylation may be an index for evaluating, for example, an extent of cell differentiation. Under these circumstances, various methods for measuring methylation have been examined.
From another viewpoint, for example, when methylation of DNA is measured in medical facilities, a rapid and accurate analysis is desired. However, conventional methods described below are not sufficient from the above viewpoints.

As known methods of analyzing DNA methylation, particularly, a method of analyzing 5-methylcytosine, there may be mentioned, for example,
(1) a method in which a DNA sample is decomposed into bases with an enzyme, and the resulting bases are analyzed by chromatography or an ELISA method,
(2) a method in which cytosine bases contained in a DNA sample are converted to uracil with an appropriate agent such as bisulfite, and a PCR is carried out using a PCR primer specifically hybridizing with a methylated DNA or a PCR primer specifically hybridizing with a unmethylated DNA,
(3) a method of directly sequencing a DNA sequence of the DNA previously treated with bisulfite, in a manner similar to that of the above method of (2), or
(4) a bisulfite-PCR-SSCP (single strand conformational polymorphism) method (non-patent reference 1). In the above method (2), the presence or absence of methylation at the region to be hybridized with each primer may be judged on the basis of an appearance of each PCR product.

These known methods can be used in analyzing methylation of a specific cytosine base, or plural cytosine bases in a specific region, but cannot be used for a comprehensive analysis of methylation of genomic DNA.

Patent reference 1 discloses a DNA array capable of comprehensively analyzing sites to be methylated in genomic DNA. Hereinafter, the site to be methylated is referred to as a methylation site, and the site which is actually methylated is referred to as a methylated site. The term "DNA array" as used herein includes, for example, a DNA microarray, a DNA chip, or a biochip.

Patent reference 1 discloses a DNA array in which plural kinds of DNA fragments (or chemically synthesized oligonucleotide probes having partial nucleotide sequences thereof) obtained by digesting genomic DNA with a methylation-insensitive restriction enzyme (for example, XmaI), in which a methylation-sensitive restriction enzyme (for example, SmaI) capable of digesting the same recognition site is known, are independently immobilized on a substrate, and a method of detecting methylation in methylated sites using the same. The term "methylation-insensitive restriction enzyme" as used herein means a restriction enzyme capable of digesting a recognition site regardless of the presence or absence of methylation in the recognition site. The term "methylation-sensitive restriction enzyme" as used herein means a restriction enzyme in which the digestion activity is affected by the presence or absence of methylation in the recognition site.

The detection method disclosed in patent reference 1 comprises the steps of:
(1) digesting genomic DNA to be analyzed with a methylation-sensitive restriction enzyme (such as SmaI, which generates a blunt end and cannot digest a recognition sequence when methylated cytosine is contained in the recognition sequence),
(2) digesting the resulting genomic DNA of the above step (1) with a methylation-insensitive restriction enzyme capable of digesting the same recognition site but generating a different cleavage end (such as XmaI, which generates a cohesive end having a 5' overhang),
(3) specifically amplifying only DNA fragments having cleavage ends generated by the methylation-insensitive restriction enzyme at both ends, and
(4) detecting the amplified DNA fragments using the DNA array. As the method of specifically amplifying only the specific DNA fragments (i.e., DNA fragments having cleavage ends generated by the methylation-insensitive restriction enzyme at both ends) in the above step (3), a method in which an XmaI adaptor is ligated with the DNA fragment and a PCR method is carried out using a primer having a partial nucleotide sequence of the adaptor is disclosed.

In the detection method disclosed in patent reference 1, since genomic DNA is digested with a methylation-sensitive restriction enzyme (the recognition cannot be digested when methylated cytosine is contained in the recognition sequence) followed by a methylation-insensitive restriction enzyme, the recognition site digested with the methylation-insensitive restriction enzyme always contains methylated cytosine. Both ends of DNA fragments amplified in the step (3) are derived from the recognition site containing methylated cytosine, i.e., methylated methylation site (methylated site), and only DNA fragments interposed between methylated sites are detected.

As above, in the detection method disclosed in patent reference 1, only DNA fragments in which both methylation sites at both ends are methylated can be detected, but DNA fragments in which either methylation site is methylated cannot be detected, and thus, there is a possibility that a slight change of methylation in a living body is overlooked. For example, it is frequently observed in cancer cells that CpG islands having a low methylation ratio in general are hypermethylated. Such a slightly increased methylation in a DNA fragment not methylated in general (for example, methylation in either end of a DNA fragment) cannot be detected by the detection method disclosed in patent reference 1.

In the detection method disclosed in patent reference 1, since the two-step digestion with restriction enzymes is essential, the digestion takes time. Although it is preferable to carry out a desalting treatment after the digestion, because a ligation efficiency in the adaptor-ligation treatment is decreased when a salt concentration is high, but the procedure of desalting is complicated. In PCR commonly used in a DNA amplification, when DNA fragments to be amplified vary in size, it is known that shorter DNA fragments are generally amplified in preference to longer DNA fragments, and thus, it is difficult to amplify all DNA fragments at the same efficiency.

In the DNA array disclosed in patent reference 1, plural kinds of DNA fragments (or chemically synthesized oligonucleotide probes) obtained by digesting genomic DNA with a methylation-insensitive restriction enzyme (such as XmaI) are independently immobilized on a substrate.
However, each DNA fragment or oligonucleotide probe does not have a special feature.

(non-patent reference 1) M.Maekawa et al., Biochemical and Biophysical Research Communications, Netherlands, 1999, vol. 62, p. 671-676
(patent reference 1) Japanese Unexamined Patent Publication No. 2003-38183 (JP 2003-38183 A1)
NO 02/00842 A2 describes a method of obtaining a centromere nucleic acid sequence from a selected organism by preparing a first sample of genomic DNA from a selected organism, obtaining a plurality of methylated nucleic acid segments from that genomic DNA and screening said methylated nucleic acid segments to identify a centromere nucleic acid sequence. PNAS Vol. 100, No. 19, 11133-11138 (2003) deals with the extent and patterns of *Arabidopsis* heterochromatin methylation by amplifying a sequencing genomic DNA treated with bisulfite, which converts cytosine, but not mehtylcytosine, to uracil. This survey reveals unexpected asymmetries in methylation patterns with one helix strength of exhibiting higher levels of methylation. Nucleic Acid Research, 2004 Vol. 32, No. 1, 287 - 297 refers the characterisation of cytosine methylated regions and 5-cytosine DNA methyltransferase in the protozoan parasite *Entamoeba histolytica.*

### DISCLOSURE OF INVENTION

### PROBLEMS TO BE SOLVED BY THE INVENTION

An object of the present invention is to remedy the above-mentioned disadvantages of the prior art, and to provide a DNA array for analyzing a DNA methylation, which enables an exhaustive analysis of methylation sites in a DNA (for example, genomic DNA), a method of producing the DNA array, and a method of analyzing DNA methylation. The present invention may be used in analyzing various modifications in bases including cytosine in a DNA, preferably in analyzing methylation of cytosine.

More particularly, an object of the present invention is to provide an DNA array and a method of analyzing DNA methylation, which enable a detection of not only a DNA fragment in which both methylation sites located at both ends are methylated, but also a DNA fragment in which only one of the methylation sites at both ends is methylated or a DNA fragment in which no methylation sites at both ends are methylated, without complicated procedures. Another object is to provide a method of producing a DNA array, which enables a classifying of nucleic acids to be arranged on an array into appropriate groups by taking into consideration a state of methylation sites at both ends. Still another object is to provide a method of analyzing DNA methylation and a method of producing a DNA array, which enable an analyzing of a methylation state of methylation sites contained in a single-stranded region (for example, a stem and loop structure, or a triple-stranded structure formed by binding a nucleic acid to a double-stranded DNA).

### MEANS FOR SOLVING THE PROBLEMS

The above objects can be solved, according to the present invention, by a method of producing a DNA array, characterized by comprising the steps of:
(1) preparing a mixture of DNA fragments in which a modified base or a base is exposed, wherein the mixture of DNA fragments is
   (a) a mixture of DNA fragments in which a modified base or a base is exposed at a cohesive end thereof, obtained by digesting genomic DNA with a restriction enzyme which can digest a DNA regardless of the presence or absence of a modification in a recognition site to generate a cohesive end containing a modified base or a base, wherein the genomic DNA is pretreated with a nuclease capable of digesting a single-stranded DNA, before digesting the genomic DNA with the restriction enzyme, or
   (b) a mixture of single-stranded DNA fragments or partially single-stranded DNA fragments in which a modified base or a base is exposed in the single-stranded region, obtained by fragmenting genomic DNA and rendering the fragmented genomic DNAs fully or partially single-stranded, wherein the genomic DNA or the fragmented genomic DNAs are pretreated with a nuclease capable of digesting a single-stranded DNA, before rendering the fragmented genomic DNAs fully or partially single-stranded.
(2) bringing the mixture of DNA fragments obtained in step (1) into contact with an antibody specific to either the modified base or to the corresponding base, and separating the mixture into a group consisting of DNA fragments which form an immunocomplex with the antibody and another group consisting of DNA fragments which do not react with the antibody, or a group consisting of DNA fragments showing a high affinity for the antibody and another group consisting of DNA fragments showing a low affinity for the antibody,
(3) identifying all or part of DNA fragments contained in each of the DNA fragment groups, and
(4) arranging one or more nucleic acids capable of hybridizing with any one of the identified DNA fragments on a substrate,

According to still another preferred embodiment of the method of the present invention, when the mixture of DNA fragments is brought into contact with the antibody in the antibody-contact step (2), at least one antigen-antibody reaction is performed under "conditions in which a monovalent binding is dissociated and a divalent binding is maintained" to separate the mixture into a group consisting of DNA fragments capable of binding to the antibody by a divalent binding (as the group consisting of DNA fragments which form an immunocomplex with the antibody) and another group consisting of DNA fragments capable of binding to the antibody by a monovalent binding (as the group consisting of DNA fragments which do not react with the antibody).

For example, the DNA fragment mixture obtained in the preparation step, each DNA fragment having cohesive ends at both ends, may be brought into contact with an antibody specific to methylated cytosine (or an anti-cytosine antibody) under ordinary conditions of an antigen-antibody reaction, to separate a complex-forming DNA fragment group, in which the DNA fragments form immunocomplexes, from an unreacted DNA fragment group, and then, the immunocomplexes may be allowed to stand under "conditions in which a monovalent binding is dissociated and a divalent binding is maintained in an antigen-antibody reaction", to separate a group consisting of DNA fragments in which methylated cytosine (or cytosine) is contained in both of the cohesive ends (as the complex-forming DNA fragment group) and another group consisting of DNA fragments in which methylated cytosine (or cytosine) is contained in only one of the cohesive ends (as the unreacted DNA fragment group).

According to still another preferred embodiment of the method of the present invention, when the mixture of DNA fragments is brought into contact with the antibody in the antibody-contact step (2), at least one antigen-antibody reaction is performed under "conditions in which a group consisting of DNA fragments showing a high affinity can be separated from another group consisting of DNA fragments showing a low affinity, on the basis of the difference between a monovalent binding and a divalent binding" to separate the mixture into a group consisting of DNA fragments capable of binding to the antibody by a divalent binding (as the group consisting of DNA fragments showing a high affinity) and another group consisting of DNA fragments capable of binding to the antibody by a monovalent binding (as the group consisting of DNA fragments showing a low affinity).

The present invention further relates to a method of analyzing a modification in a DNA to be assayed, characterized by comprising the steps of:
(1) preparing a mixture of DNA fragments in which a modified base or a base is exposed, from the DNA to be assayed, wherein the mixture of DNA fragments is
   (a) a mixture of DNA fragments in which a modified base or a base is exposed at a cohesive end thereof, obtained by digesting genomic DNA with a restriction enzyme which can digest a DNA regardless of the presence or absence of a modification in a recognition site to generate a cohesive end containing a modified base or a base, wherein the genomic DNA is pretreated with a nuclease capable of digesting a single-stranded DNA, before digesting the genomic DNA with the restriction enzyme, or
   (b) a mixture of single-stranded DNA fragments or partially single-stranded DNA fragments in which a modified base or a base is exposed in the single-stranded region, obtained by fragmenting genomic DNA and rendering the fragmented genomic DNAs fully or partially single-stranded, wherein the genomic DNA or the fragmented genomic DNAs are pretreated with a nuclease capable of digesting a single-stranded DNA, before rendering the fragmented genomic DNAs fully or partially single-stranded.
(2) bringing the mixture of DNA fragments obtained in the step (1) into contact with an antibody specific to either the modified base or to the corresponding base, and separating the mixture into a group consisting of DNA fragments which form an immunocomplex with the antibody and another group consisting of DNA fragments which do not react with the antibody, or a group consisting of DNA fragments showing a high affinity for the antibody and another group consisting of DNA fragments showing a low affinity for the antibody, and
(3) analyzing all or part of DNA fragments contained in each of the DNA fragment groups with a DNA array obtainable by the method according to any one of claims 1 to 3.

The term "cytosine" as used herein means cytosine not methylated, i.e., unmethylated cytosine. The term "methylated cytosine" as used herein means 5-methylcytosine.

The term "antibody specific to methylated cytosine" or "anti-methylated cytosine antibody" as used herein means an antibody which specifically reacts with methylated cytosine, but does not specifically react with cytosine, unless otherwise specified. The term "antibody specific to cytosine" or "anti-cytosine antibody" as used herein means an antibody which specifically reacts with cytosine, but does not specifically react with methylated cytosine, unless otherwise specified.

The term "antibody" as used herein includes a monoclonal antibody and a polyclonal antibody. The term "antibody" includes not only an antibody in a narrow sense (i.e., an immunoglobulin molecule per se), but also a fragment of an antibody, such as Fab, Fab', F(ab')₂, or Fv.

The term "base" as used herein means a naturally-occurring or chemically-synthesized base capable of forming one or more hydrogen bonds with a nucleic acid (including a naturally-occurring DNA and RNA, and artificial modification thereof). As the base, there may be mentioned, for example, cytosine, adenine, guanine, thymine, or uracil, preferably cytosine. The term "modified base" as used herein means a modified base which may be contained in a nucleic acid. As the "modification" in the "modified base", there may be mentioned, for example, methylation, oxidation, dimerization, or alkylation, preferably methylation. As the "modified base", there may be mentioned, for example, methylated cytosine, methylated adenine, methylated guanine, oxoguanine (such as 8-oxoguanine), hydroxyadenine (such as 2-hydroxyadenine), thymidine dimer, or alkylated guanine, preferably methylated cytosine.
For example, oxoguanine or thymidine dimer is a change undesirable to an organism, and the present invention may be used in analyzing an oxidative modification of guanine or a generation of thymidine dimer. Further, the presence or absence of alkylated guanine may be used as an index of the influence of alkylated agents known as mutagens. Furthermore, 8-oxoguanine or 2-hydroxyadenine is a modified base which is generated when genomic DNA is damaged by active oxygen, and thus, the influence of active oxygen may be analyzed by detecting the presence or absence of 8-oxoguanine or 2-hydroxyadenine.

Hereinafter, the DNA array for analyzing a DNA modification, the method of producing the same, and the method of analyzing a DNA modification according to the present invention will be mainly explained with respect to a case in which the base and the modified base are cytosine and methylated cytosine, respectively, as a DNA array for analyzing DNA methylation, a method of producing the same, and a method of analyzing DNA methylation. However, the present invention is not limited to the case in which the base and the modified base are cytosine and methylated cytosine, respectively, and the present invention in which another base and other modified base are used may be carried out for those skilled in the art, in accordance with the disclosures as described herein and technical common knowledge.

### EFFECTS OF THE INVENTION

According to the method of the present invention for analyzing methylation, on the basis of trends of positive spots and/or negative spots on the DNA array (i.e., numbers thereof) in each cell to be analyzed, the methylation ratio in genomic DNA of the cell can be evaluated. Further, on the basis of profiles of positive spots and/or negative spots, the state of the cell (for example, malignancy of a cancer cell, differentiation of a cell, or morbidity of diseases) can be evaluated.

According to the production method of the present invention, the DNA array of the present invention, which may be used in the method of the present invention for analyzing methylation, can be produced. According to the production method of the present invention, DNA fragments obtained by digesting genomic DNA from a cell to be analyzed with an appropriate methylation-insensitive restriction enzyme can be classified into DNA fragments in which both methylation sites at both ends are methylated, DNA fragments in which either methylation site at both ends is methylated, and DNA fragments in which both methylation sites are not methylated, and a DNA array in which the classified DNA fragments are immobilized on a substrate can be produced. According to the DNA array of the present invention, a slight change in methylation, for example, methylation at either methylation site at both ends, can be detected.

According to the present invention, the state of methylation at one or more methylation sites in a single-stranded region (for example, a stem and loop structure, or a triple-stranded structure formed by binding a nucleic acid to a double-stranded DNA) may be analyzed. Hitherto, not only has such a method of analyzing methylation in a single-stranded region not been known, but also the object has never been recognized. According to the present invention, the DNA array of the present invention and the method of the present invention for analyzing methylation, which may be used in detecting the state of methylation in a single-stranded region without complicated procedures, can be provided.

### BRIEF DESCRIPTION OF THE DRAWINGS

Figure 1 schematically illustrates the state of a monovalent binding in which an antibody molecule binds to a double-stranded DNA via one antigen-binding site.
Figure 2 schematically illustrates the state of a divalent binding in which an antibody molecule binds to a double-stranded DNA via two antigen-binding sites.
Figure 3 schematically illustrates the state of a divalent binding in which each of two antibody molecules immobilized on a carrier binds to a double-stranded DNA via one antigen-binding site.
Figure 4 schematically illustrates the state in which each of two antibody molecules binds to a double-stranded DNA via one antigen-binding site.
Figure 5 schematically illustrates each step of the MONIC method.
Figure 6 schematically illustrates each step of the MONIC-Loop Trap method.
Figure 7 shows the result of electrophoresis of DNA fragments contained in the adsorption fraction obtained from a DNA fragment mixture by the MONIC method.

### BEST MODE FOR CARRYING OUT THE INVENTION

### [1] DNA array and method of producing the same according to the present invention

The method of the present invention for producing the DNA array comprises the preparation step, the antibody-contact step, the identification step, and the arrangement step.
On the basis of differences in specificities of antibodies used in the antibody-contact step, the method of the present invention for producing the DNA array includes a method (hereinafter referred to as methylated cytosine-type production method) of producing a DNA array (hereinafter referred to as methylated cytosine-type array) which can be used in analyzing DNA fragments in which at least one methylated cytosine is exposed, and a method (hereinafter referred to as cytosine-type production method) of producing a DNA array (hereinafter referred to as cytosine-type array) which can be used in analyzing DNA fragments in which at least one cytosine is exposed.
Further, the method of the present invention for producing the DNA array includes, on the basis of types of DNA fragment mixtures used, an embodiment [hereinafter referred to as modified nucleotide immunocapturing method (MONIC method)] using the above-mentioned DNA fragment mixture (a) or the DNA fragment mixture (b), and an embodiment [hereinafter referred to as modified nucleotide immunocapturing/loop-trap method; MONIC-Loop Trap method] using the above-mentioned DNA fragment mixture (c).

In the methylated cytosine-type production method, included in the production method of the present invention, at least an anti-methylated cytosine antibody (i.e., an antibody which specifically reacts with methylated cytosine, but does not specifically react with cytosine) is used as the antibody used in the antibody-contact step.
In the cytosine-type production method, included in the production method of the present invention, at least an anti-cytosine antibody (i.e., an antibody which specifically reacts with cytosine, but does not specifically react with methylated cytosine) is used as the antibody used in the antibody-contact step.
In this connection, the anti-methylated cytosine antibody and anti-cytosine antibody may be used as a combination thereof in the methylated cytosine-type or cytosine-type production method.
Hereinafter, the methylated cytosine-type production method and the methylated cytosine-type DNA array will be explained, and then, the cytosine-type production method and the cytosine-type DNA array will be explained.

### (1) Methylated cytosine-type production method and Methylated cytosine-type DNA array

In the preparation step of the methylated cytosine-type production method of the present invention, an appropriate DNA material is used to prepare a DNA fragment mixture in which one or more methylated cytosines or one or more cytosines to be methylated are exposed in each DNA fragment.
The DNA material used in the preparation step is not particularly limited, so long as it may be analyzed by the method of analyzing methylation of the present invention, that is, it is a DNA which may contain one or more methylated cytosines or one or more cytosines to be methylated. As the DNA material, there may be mentioned, for example, genomic DNA in a cell (for example, an animal cell or a plant cell), or a mixture of free DNA fragments contained in a biological sample or a sample derived therefrom (for example, blood, plasma, serum, urine, lymph, a spinal fluid, saliva, a ascites fluid, an amniotic fluid, mucus, milk, bile, gastric juices, or an artificial dialysis fluid after dialysis).

When genomic DNA in a cell is used, it is preferable to use a cell having a high methylation ratio, because DNA fragments to be arranged on a DNA array are selected on the basis of the presence or absence of methylated cytosine in the methylated cytosine-type production method. When the cell having a high methylation ratio is used, the DNA fragment mixture obtained in the preparation step may contain a high content of DNA fragments in which at least one methylated cytosine is exposed.
As the cell having a high methylation ratio, a cell having a methylation ratio higher than that in a normal cell, for example, cancer cells, a sperm, or seeds of plants, may be used. Only one kind of cell may be used alone, or plural kinds of cells may be used as a combination thereof. In this connection, since a methylation ratio of a normal cell varies in accordance with various conditions, for example, the type of a tissue or organ, aging, external factors, a developmental stage of an individual, differentiation, or de-differentiation, it is preferable to select an appropriate cell used in the preparation stage, in accordance with an intended purpose of a DNA array, particularly the type of cells to be assayed.

When genomic DNA of a cell is used as a DNA material in the preparation step of the methylated cytosine-type production method, the mixture of DNA fragments in which methylated cytosine or cytosine to be methylated is exposed may be prepared from the genomic DNA by, for example, (a) digesting the genomic DNA with a restriction enzyme which can digest a DNA regardless of the presence or absence of methylation in a recognition site (i.e., methylation-insensitive) to generate a cohesive end containing methylated cytosine or cytosine to be methylated (hereinafter referred to as C-containing cohesive end), or (b) fragmenting the genomic DNA and rendering the fragmented genomic DNAs fully or partially single-stranded to prepare a mixture of single-stranded DNA fragments or partially single-stranded DNA fragments.

When the methylation-insensitive restriction enzyme capable of generating a C-containing cohesive end is used in the preparation step, the genomic DNA can be fragmented with the restriction enzyme to generate cohesive ends and, simultaneously, methylated cytosine or cytosine to be methylated can be exposed at the cohesive ends.
Since the C (cytosine) in a 5'-CG-3' sequence is methylated in higher animals, it is preferable to use a methylation-insensitive restriction enzyme which generates a cohesive end containing the CG sequence (hereinafter referred to as CG-containing cohesive end). As such a restriction enzyme, for example, restriction enzymes listed in Table 1 may be used. In Table 1, the underlined "C" means cytosine to be methylated, and the symbol ":" means a cleavage site. When a base represented by the symbol "Y" or "N" not underlined is C, there is a possibility that the C may be methylated. For example, a restriction enzyme BsaWI cleaves between "W" and "C" of a recognition sequence "WCCGGW" to generate a 5'-cohesive end of a 5'-CCGG-3' sequence. Further, a restriction enzyme MspI cleaves between "C" and "C" of a recognition sequence "CCGG" to generate a 5'-cohesive end of a 5'-CG-3' sequence. In this connection, it is not necessary that the whole of the CG sequence is exposed in a single-stranded region of a cohesive end, so long as at least one cytosine to be methylated contained in the CG sequence is located on the single-stranded region.

**Table 1**

| Restriction enzyme | Recognition sequence and cleavage site | Symbol |
|---|---|---|
| BsaWI | W:CCGGW | W = A or T |
| BsoBI | C:YCGRG | Y = C or T, R = A or G |
| BssSI | C:TCGTG | |
| MspI | C:CGG | |
| TaqI | T:CGA | |
| XmaI | C:CCGGG | |
| BsaJI | C:CNNGG | N = arbitrary base |
| PspAI | C:CCGGG | |

Since the C (cytosine) in a 5'-CNG-3' sequence is methylated in plants, it is preferable to use a methylation-insensitive restriction enzyme which generates a cohesive end containing the CNG sequence (hereinafter referred to as CNG-containing cohesive end). As such a restriction enzyme, for example, restriction enzymes listed in Table 2 may be used. In Table 2, the underlined "C" means cytosine to be methylated, and the symbol ":" means a cleavage site. When a base represented by the symbol "Y" or "N" not underlined is C, there is a possibility that the C may be methylated. In this connection, it is not necessary that the whole of the CNG sequence be exposed in a single-stranded region of a cohesive end, so long as at least one cytosine to be methylated contained in the CNG sequence is located on the single-stranded region.

**Table 2**

| Restriction enzyme | Recognition sequence and cleavage site | Symbol |
|---|---|---|
| BsaWI | W:CCGGW | W = A or T |
| MspI | C:CGG | |
| XmaI | C:CCGGG | |
| BsoBI | C:YCGRG | Y = C or T, R = A or G |
| BsaJI | C:CNNGG | N = arbitrary base |
| PspAI | C:CCGGG | |

When the DNA fragment mixture is prepared using the methylation-insensitive restriction enzyme capable of generating a C-containing cohesive end, a restriction enzyme not generating a cohesive end with methylated cytosine [for example, a restriction enzyme not generating a C-containing cohesive end (for example, a restriction enzyme capable of generating a blunt end, or a resection enzyme capable of generating a cohesive end without C)] may be used together therewith, if desired. When the restriction enzyme not generating a cohesive end with methylated cytosine is used together therewith, an additional end newly generated with the enzyme is a blunt end, or a cohesive end without methylated cytosine, and thus, does not affect the analysis of methylation in a C-containing cohesive end.

When the mixture of single-stranded DNA fragments or partially single-stranded DNA fragments is prepared from genomic DNA in the preparation step, an order of the step of fragmenting genomic DNA and the step of rendering genomic DNA single-stranded is not particularly limited. Either step may be carried out prior to the other, or both steps may be performed simultaneously.

As a method of fragmenting genomic DNA, there may be mentioned, for example, a method using a restriction enzyme (including a restriction enzyme capable of generating a blunt end and a restriction enzyme capable of generating a cohesive end), a method of physically cleaving genomic DNA (for example, ultrasonication, ultraviolet irradiation, radiation irradiation, electron beam irradiation), or a method of chemically cleaving genomic DNA.

When a DNA (including genomic DNA and fragmented DNAs) is rendered single-stranded, the full-length of the DNA, or only a partial portion thereof may be rendered single-stranded. The term "single-stranded" as used herein means the state in which the full-length or a partial portion of one DNA chain which forms a double-stranded DNA does not hybridize with the other DNA chain (i.e., complementary chain) and one or more bases are exposed. For example, the "single-stranded" structure includes, for example, a structure consisting of only a complete single-stranded DNA, a triple-stranded structure described below, and a structure in which one or plural bases are exposed by a mismatch of one or plural base pairs.

As a method of rendering the full-length of a DNA single-stranded, there may be mentioned, for example, a thermal denaturation or a treatment with a denaturing agent.

As a method of rendering a partial portion of a DNA single-stranded, there may be mentioned, for example, a method of providing a nucleic acid (for example, an oligonucleotide or a polynucleotide; hereinafter referred to as nucleic acid for single-stranded rendering) capable of hybridizing with one chain contained in a DNA to be rendered single-stranded. When a double-stranded DNA to be rendered single-stranded is brought into contact with the nucleic acid for single-stranded rendering, a specific portion of one chain of the double-stranded DNA is hybridized with the nucleic acid for single-stranded rendering to form a double-stranded structure. At the same time, a specific portion of the other chain of the double-stranded DNA is rendered single-stranded, and as a result, a triple-stranded structure is formed.
The nucleic acid for single-stranded rendering may be designed to target a portion containing one or more methylated cytosines or cytosines to be methylated in a DNA to be rendered single-stranded. Only one kind of nucleic acid may be used alone, or plural kinds of nucleic acids may be used as a combination thereof. It is preferable to use the nucleic acid for single-stranded rendering, because methylated cytosine of cytosine to be methylated in a desired specific portion can be selectively exposed.

In some DNA fragments, one or more partial portions thereof are rendered single-stranded without an artificial treatment for single-stranded treatment (such as an addition of the nucleic acid for single-stranded rendering), because of, for example, partially forming a stem and loop structure, or a triple-stranded structure in which a nucleic acid (such as an RNA or a DNA) binds to a double-stranded DNA. When such DNA fragments are used as a DNA material, the DNA fragments without such an artificial treatment may be used in the subsequent antibody-contact step, as the mixture of DNA fragments in which methylated cytosine or cytosine to be methylated is exposed.
When genomic DNA is used as a DNA partially having one or more single-stranded regions, the genomic DNA is fragmented with an appropriate restriction enzyme, and the resulting fragmented genomic DNA may be used in the subsequent antibody-contact step. When, as the restriction enzyme, a restriction enzyme not generating a cohesive end with methylated cytosine [for example, a restriction enzyme not generating a C-containing cohesive end (for example, a restriction enzyme capable of generating a blunt end, or a resection enzyme capable of generating a cohesive end without C)] is used, an analysis of methylation in one or more single-stranded regions which are originally located in the genomic DNA before treating with the restriction enzyme is not affected. If desired, the DNA may be used in the subsequent antibody-contact step, after further forming one or more artificial single-stranded regions (for example, a cohesive end or a triple-stranded structure) in addition to the original single-stranded region(s).

When the mixture of free DNA fragments contained in a biological sample or a sample derived therefrom is used as a DNA material in the preparation step of the methylated cytosine-type production method, the mixture of DNA fragments in which methylated cytosine or cytosine to be methylated is exposed may be prepared by, for example, a method using the methylation-insensitive restriction enzyme capable of generating a C-containing cohesive end (preferably, a CG-containing cohesive end or a CNG-containing cohesive end), or a method using the nucleic acid for single-stranded rendering. When the free DNA fragments are rendered fully or partially single-stranded, the DNA fragments without an artificial treatment for single-stranded treatment may be used in the subsequent antibody-contact step, as the mixture of DNA fragments in which methylated cytosine or cytosine to be methylated is exposed.

When the mixture of free DNA fragments contained in a biological sample or a sample derived therefrom is used as a DNA material, an amount of the free DNA fragments contained in the sample is sometimes insufficient to carry out the following steps. In such a case, a sufficient amount of the DNA fragments may be obtained by performing a DNA amplification, such as a PCR. For example, after a mixture of free DNA fragments is purified from the sample and ligated with an appropriate adaptor, a DNA amplification may be carried out using a primer specific to the adaptor.

In the antibody-contact step of the methylated cytosine-type production method, the DNA fragment mixture obtained in the preparation step is brought into contact with an anti-methylated cytosine antibody to separate a group consisting of DNA fragments which form an immunocomplex with the antibody (i.e., complex-forming DNA fragment group) from a group consisting of DNA fragments which do not react with the antibody (i.e., unreacted DNA fragment group).

The anti-methylated cytosine antibody may be prepared by using methylated cytosine as an antigen in accordance with a conventional method [for example, Japanese Unexamined Patent Publication No. 2004-347508 (JP 2004-347508 A1)], or a commercially available antibody (for example, anti-5-methylcytosine monoclonal antibody, cat#01519721; Wako Pure Chemical Industries, Osaka) may be used. For example, 5-methylcytidine is bound to keyhole limpet hemocyanin to prepare an immunogen, and the immunogen is administered to a mouse to obtain the antibody of interest.

The method of bringing the DNA fragment mixture into contact with the anti-methylated cytosine antibody is not particularly limited, so long as the complex-forming DNA fragment group may be separated from the unreacted DNA fragment group. The complex-forming DNA fragment group may be separated from the unreacted DNA fragment group, for example, by bringing the DNA fragment mixture into contact with the anti-methylated cytosine antibody carried on an appropriate carrier. Alternatively, after the DNA fragment mixture is brought into contact with the anti-methylated cytosine antibody, the complex-forming DNA fragment group is purified from the whole in accordance with a conventional method to separate the complex-forming DNA fragment group from the unreacted DNA fragment group. In the former method using an appropriate carrier, the complex-forming DNA fragment group may be separated from the unreacted DNA fragment group, for example, by an affinity chromatography using the anti-methylated cytosine antibody, or an antigen-antibody reaction using the anti-methylated cytosine antibody carried on particles separable by centrifugation or magnetic particles. In the latter two-step method, the complex-forming DNA fragment group may be purified, for example, by using a column or particles (such as magnetic particles) carrying protein A or protein G thereon.

Among the mixture of DNA fragments in which methylated cytosine or cytosine to be methylated is exposed prepared in the preparation step, DNA fragments in which methylated cytosine is exposed form an immunocomplex together with the anti-methylated cytosine antibody, and are separated as the complex-forming DNA fragment group. DNA fragments in which methylated cytosine is not exposed do not react with the anti-methylated cytosine antibody, and are separated as the unreacted DNA fragment group.

When the DNA fragment mixture is prepared using the methylation-insensitive restriction enzyme capable of generating a C-containing cohesive end in the preparation step, the DNA fragments have C-containing cohesive ends at both ends. When the complex-forming DNA fragment group is separated from the unreacted DNA fragment group by contact with the anti-methylated cytosine antibody, the DNA fragments contained in the complex-forming DNA fragment group include a DNA fragment in which both methylation sites located at both ends are methylated (i.e., methylated cytosine exists at both C-containing cohesive ends) and a DNA fragment in which only either methylation site at both ends is methylated (i.e., methylated cytosine exists at only either of two C-containing cohesive ends).

In this case (i.e., when the DNA fragment mixture is prepared using the methylation-insensitive restriction enzyme capable of generating a C-containing cohesive end), the contact of the DNA fragment mixture with the anti-methylated cytosine antibody may be carried out under the following specific conditions to separate a complex-forming DNA fragment group consisting of only DNA fragments in which both methylation sites located at both ends are methylated (i.e., methylated cytosine exists at both C-containing cohesive ends) from an unreacted DNA fragment group.

It is known that a divalent binding of an antibody shows a higher affinity than a monovalent binding by a factor of approximately 10³ (M⁻¹) in an antigen-antibody reaction [for example, Ivan Roitt, Jonathan Brostoff, and David Male; Tomio Tada, trans-ed., Menekigaku Illustrated (original, 5th ed.), February 10, 2000, Nankodo, p. 110 (original title: IMMUNOLOGY, FIFTH EDITION)]. The term "divalent binding" as used herein means that one antibody molecule (or two antibody molecules immobilized on a carrier) binds to an antigen at two antigen-binding sites. The term "monovalent binding" as used herein means that one antibody molecule binds to an antigen at one antigen-binding site.

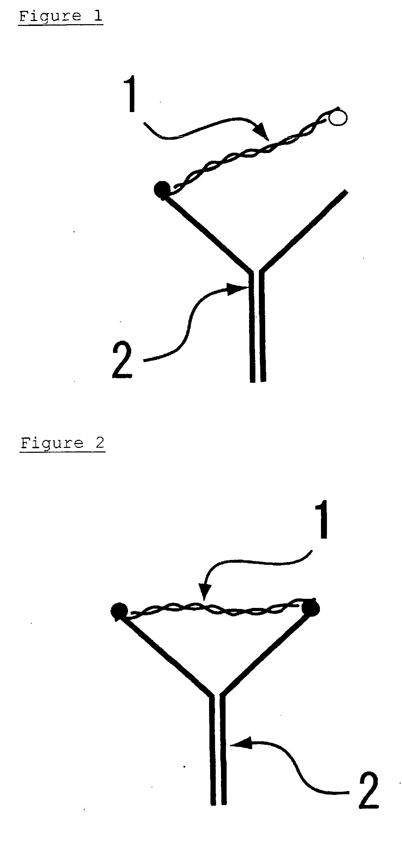

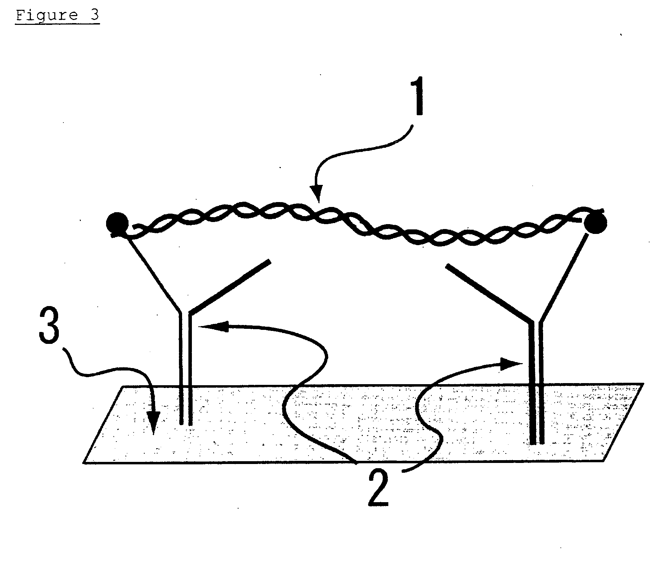

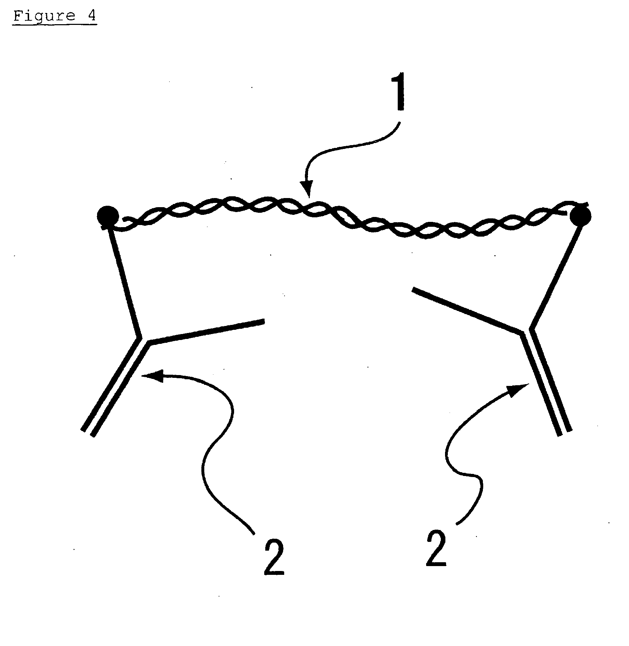

Hereinafter, the divalent binding and the monovalent binding will be further illustrated with reference to Figures 1 to 4. Figures 1 to 4 schematically illustrate the binding between a molecule of double-stranded DNA (1) having cohesive ends at both ends and one or two molecules of anti-methylated cytosine antibody (2). The black circle (in Figures 1 to 4) and the white circle (in Figure 1) at cohesive ends of the double-stranded DNA (1) represent methylated cytosine and cytosine, respectively.
As shown in Figures 1 to 4, a molecule of antibody (2) has two antigen-binding sites. In the monovalent binding, the antibody (2) binds to the double-stranded DNA (1) as the antigen at either antigen-binding site (Figure 1). In the divalent binding, a molecule of antibody (2) binds to a molecule of antigen (1) at two antigen-binding sites (Figure 2), or each of two antibody molecules (2) immobilized on a carrier (3) binds to an antigen molecule (1) at one antigen-binding site (Figure 3). In Figure 4, each of two antibody molecules (2) binds to an antigen molecule (1) at one antigen-binding site, and it is known that the affinity in this case is the same as that of the monovalent binding.

As described above, the affinity of the divalent binding is different from that of the monovalent binding, and thus, it is possible to dissociate the monovalent binding and maintain the divalent binding by selecting appropriate conditions in an antigen-antibody reaction system. The binding between an antigen and an antibody is based on, for example, an electrostatic bond, a hydrogen bond, or a hydrophobic bond. It is known that the electrostatic bond may be affected by, for example, a salt concentration or a pH. Similarly, it is known that the hydrogen bond and the hydrophobic bond may be affected by, for example, a concentration of urea or guanidine hydrochloride and a concentration of polyethylene glycol, respectively.

For example, with respect to the salt concentration (for example, an NaCl concentration) in an antigen-antibody reaction system, conditions in which the divalent binding is maintained but the monovalent binding is excluded (including conditions in which the divalent binding and the monovalent binding coexist and the divalent binding dominates over the monovalent binding with respect to the ratio) vary in accordance with the kind of an antibody or a salt. The salt concentration may be easily determined in accordance with an antibody used in an antigen-antibody reaction, for example, by using experimental systems in Examples described below.
Similarly, with respect to conditions other than the salt concentration (for example, a concentration of urea, guanidine hydrochloride, or polyethylene glycol, or a pH) capable of affecting the affinity, the conditions in which the divalent binding is maintained but the monovalent binding is excluded may be easily determined in accordance with an antibody used in an antigen-antibody reaction.

When a combination of an anti-methylated cytosine antibody and an anti-cytosine antibody is used in the antibody-contact step, a DNA fragment group consisting of only DNA fragments in which only either methylation site (i.e., cytosine) at both ends is methylated (i.e., methylated cytosine exists at only either of two C-containing cohesive ends) may be obtained as the complex-forming DNA fragment group.
For example, the DNA fragment mixture obtained in the preparation step is brought into contact with the anti-methylated cytosine antibody to separate a group consisting of DNA fragments which form an immunocomplex with the anti-methylated cytosine antibody (a complex-forming DNA fragment group) from a group consisting of DNA fragments which do not react with the anti-methylated cytosine antibody (an unreacted DNA fragment group). Then, the obtained complex-forming DNA fragment group is brought into contact with the anti-cytosine antibody to separate a group consisting of DNA fragments which form an immunocomplex with the anti-cytosine antibody (another complex-forming DNA fragment group) from a group consisting of DNA fragments which do not react with the anti-cytosine antibody (another unreacted DNA fragment group). The DNA fragment group of interest may be obtained as the another complex-forming DNA fragment group. In this connection, the contact order of the anti-methylated cytosine antibody and the anti-cytosine antibody may be reversed.
Alternately, after the antibody-contact step may be carried out using the anti-methylated cytosine antibody under ordinary conditions, an elution may be carried out under conditions in which the monovalent binding is dissociated and the divalent binding is maintained in an antigen-antibody reaction to obtain the DNA fragment group consisting of only DNA fragments in which only either methylation site (i.e., cytosine) at both ends is methylated.
The term "ordinary conditions in an antigen-antibody reaction" as used herein means conditions commonly used in an antigen-antibody reaction, particularly conditions in which the monovalent binding is maintained.

As described above, one or more DNA fragment groups different in the state of methylation of cytosine contained in the C-containing cohesive ends at both ends thereof may be obtained in the antibody-contact step, by determining conditions of an antigen-antibody reaction, or selecting one or more antibodies or a combination thereof.
For example, a DNA fragment group consisting of DNA fragments in which at least one methylation site (i.e., cytosine) at both ends is methylated (i.e., methylated cytosine exists in at least one of two C-containing cohesive ends) may be obtained as the complex-forming DNA fragment group by carrying out an antigen-antibody reaction using the anti-methylated cytosine antibody under ordinary conditions. For example, a DNA fragment group consisting of DNA fragments in which both methylation sites (i.e., cytosine) at both ends are methylated (i.e., methylated cytosine exists at both C-containing cohesive ends) may be obtained as the complex-forming DNA fragment group by carrying out an antigen-antibody reaction using the anti-methylated cytosine antibody under conditions in which the monovalent binding is dissociated and the divalent binding is maintained.
For example, a DNA fragment group consisting of DNA fragments in which either methylation site (i.e., cytosine) at both ends is methylated (i.e., methylated cytosine exists at either of two C-containing cohesive ends) may be obtained as the complex-forming DNA fragment group by carrying out an antigen-antibody reaction using a combination of the anti-methylated cytosine antibody and the anti-cytosine antibody under conditions in which the monovalent binding is dissociated and the divalent binding is maintained.

In the antibody-contact step of the methylated cytosine-type production method, the complex-forming DNA fragment may be separated from the unreacted DNA fragment group, as described above, and the group consisting of DNA fragments showing a high affinity for the antibody (high affinity DNA fragment group) may be separated from the group consisting of DNA fragments showing a low affinity for the antibody (low affinity DNA fragment group). For example, it is well-known in an affinity chromatography using an antibody-immobilized column (i.e., antibody affinity column) that a solution, which is prepared by dissolving an antigen mixture in a mobile phase (for example, a buffer containing a specific concentration of salt commonly used to dissociate an immunocomplex) capable of providing affinities between the antibody and antigens, is passed through the antibody-immobilized carriers to separate the antigens on the basis of differences in affinities thereof [Japanese Biochemical Society ed., Zoku Seikagaku Jikken Koza 5, Meneki Seikagaku Jikken hou, (1986), Tokyo Kagaku Dojin (36-7, 3-chome, Sengoku, Bunkyo-ku, Tokyo); and Current Protocols in Immunology, WILEY, 8.2.1-8.2.5].

In the present invention, a DNA fraction of interest may be obtained by determining the conditions of a chromatography (particularly, conditions for an affinity) in accordance with, for example, an antibody, a carrier, and a DNA mixture to be separated. The DNA fragment mixture obtained in the preparation step is dissolved in a mobile phase capable of providing appropriate conditions, and the solution is passed through an affinity column immobilized with the anti-methylated cytosine antibody, to separate, for example, a group consisting of DNA fragments capable of divalently binding to the antibody (as the high affinity DNA fragment group) from a group consisting of DNA fragments capable of monovalently binding to the antibody (as the low affinity DNA fragment group).

As a factor which affects an affinity, there may be mentioned, for example, a pH, or the kind or concentration of a compound contained in the mobile phase [for example, a salt (for example, sodium chloride), glycerin, polyethylene glycol, thiocyanate (for example, sodium thiocyanate or potassium thiocyanate), urea, or a hapten].

For example, when sodium chloride is used as a salt, it may be carried out at a concentration of generally 0.05 to 3 mol/L, preferably 0.15 to 2 mol/L. When glycerin is used, it may be carried out at a concentration of generally 0 to 50%, preferably 0 to 30%. When polyethylene glycol is used, it may be carried out at a concentration of generally 0 to 50%, preferably 0 to 20%. When thiocyanate is used, it may be carried out at a concentration of generally 0 to 3 mol/L, preferably 0 to 1 mol/L. When urea is used, it may be carried out at a concentration of generally 0 to 4 mol/L, preferably 0 to 2 mol/L, more preferably 0 to 1 mol/L. When a hapten (for example, methylated cytosine when the anti-methylated cytosine is used) is used, it may be carried out at a concentration of generally 0 to 100 mmol/L, preferably 0 to 20 mmol/L. With respect to the concentrations of these compounds, it may be carried out at a constant concentration, or the concentration may be changed continuously (i.e., gradient elution) or discontinuously (i.e., stepwise elution).

It may be carried out at, generally pH 2 to 11.5, preferably pH 4 to 9, more preferably pH 6 to 8. It may be carried out by a pH gradient, for example, a gradient from pH 7.5 (when binding) to pH 4, or a gradient from pH 7.5 (when binding) to pH 11.5. More particularly, it may be carried out by, for example, a gradient from 10 mmol/L phosphate buffer (pH 7.5)/0.15 mol/L NaCl to 10 mmol/L acetate buffer (pH 4.0)/0.15 mol/L NaCl, or a gradient from 10 mmol/L phosphate buffer (pH 7.5)/0.15 mol/L NaCl to 50 mmol/L N-triethanolamine (pH 11.5)/0.15 mol/L NaCl.

In the identification step of the methylated cytosine-type production method, all or part of DNA fragments contained in each of the DNA fragment groups (preferably complex-forming DNA fragment group) obtained in the antibody-contact step are identified. In the identification step, information of each DNA fragment is obtained so that one or more nucleic acids used in the subsequent arrangement step may be prepared. In general, it is preferable to determine the nucleotide sequence of each DNA fragment so that the nucleic acids may be designed. Each DNA fragment cloned or the DNA fragment group per se may be used to determine each nucleotide sequence in accordance with a conventional method.

When the antibody-contact step is carried out under conditions in which the monovalent binding is dissociated and the divalent binding is maintained in an antigen-antibody reaction, the complex-forming DNA fragment group obtained in the antibody-contact step consists of DNA fragments in which both methylation sites at both ends are methylated (i.e., methylated cytosine exists at both C-containing cohesive ends) (Figure 2 or Figure 3). In this case, information of DNA fragments in which both methylation sites at both ends are methylated may be obtained in the identification step.
In this connection, when the antigen-antibody reaction under the specific conditions is carried out using an antibody not immobilized on a carrier, the divalent binding may be maintained only in the case shown in Figure 2, and only information of DNA fragments in which both methylation sites at both ends are methylated and the base length is approximately 40 bp (corresponding to a distance between the antigen-binding sites of an immunoglobulin molecule) may be obtained.

When the antigen-contact step is carried out under ordinary conditions, the complex-forming DNA fragment group obtained in the antigen-contact step contains DNA fragments in which both methylation sites at both ends are methylated (i.e., methylated cytosine exists at both C-containing cohesive ends) and DNA fragments in which either methylation site at both ends is methylated (i.e., methylated cytosine exists at only either of two C-containing cohesive ends). In this case, if the complex-forming DNA fragment group per se is used in the identification step, information of each DNA fragment may be obtained, but it is impossible to determine which DNA fragment is a DNA fragment in which both methylation sites at both ends are methylated, or a DNA fragment in which either methylation site at both ends is methylated.

In the identification step, DNA fragments in which both methylation sites at both ends are methylated may be separated from DNA fragments in which either methylation site at both ends is methylated, on the basis of the difference in affinity between the divalent binding and the monovalent binding, to obtain information of the DNA fragments in which either methylation site at both ends is methylated. For example, after the antibody-contact step is carried out under ordinary conditions, an elution is carried out under conditions in which the monovalent binding is dissociated and the divalent binding is maintained in an antigen-antibody reaction. The eluted DNA fragments may be identified to obtain information of the DNA fragments in which either methylation site at both ends is methylated.

In the arrangement step of the methylated cytosine-type production method, one or more nucleic acids (for example, oligonucleotides or polynucleotides) capable of hybridizing with any one of the DNA fragments identified in the identification step are arranged on a substrate.
The nucleic acids may be designed and prepared on the basis of information of each DNA fragment identified in the identification step, in accordance with conventional methods for producing a DNA array.
For example, it is preferable to use oligonucleotides chemically synthesized on the basis of the nucleotide sequence of each DNA fragment, because melting temperatures (Tm) of all nucleic acids arranged on a substrate can match closely. Alternately, DNA fragments per se cloned in the identified step, or partial fragments per se derived therefrom may be arranged on a substrate. DNA fragments per se contained in the complex-forming DNA fragment group obtained in the antibody-contact step, or DNAs obtained by amplifying the DNA fragments may be arranged on a substrate.

The nucleic acids may be arranged on a substrate in accordance with conventional methods for producing a DNA array. As a method of arranging the nucleic acids on a substrate, there may be mentioned, for example, a method of directly synthesizing oligonucleotides on a substrate, or a method of spotting previously prepared nucleic acids on a substrate to immobilize the nucleic acids thereon via, for example, covalent bonds or ionic bonds.

In a DNA array produced by the methylated cytosine-type production method of the present invention (i.e., the methylated cytosine-type DNA array of the present invention), one or more nucleic acids capable of hybridizing with DNA fragments (among the DNA fragments contained in the DNA fragment mixture prepared in the preparation step) containing methylated cytosine in a specific region (i.e., DNA fragments in which at least one cytosine in a specific region is methylated) are arranged on a substrate. For example, when the methylation-insensitive restriction enzyme capable of generating a C-containing cohesive end is used, the specific region is the C-containing cohesive ends at both ends. When the nucleic acid for single-stranded rendering is used, the specific region is a target region of the nucleic acid for single-stranded rendering. In a DNA fragment containing a stem and loop structure, the specific region is a loop region.

More particularly, in a DNA array produced by the methylated cytosine-type production method of preparing the DNA fragment mixture using the methylation-insensitive restriction enzyme capable of generating a C-containing cohesive end, one or more nucleic acids capable of hybridizing with DNA fragments containing methylated cytosine in at least one of C-containing cohesive ends at both ends (i.e., DNA fragments in which at least one cytosine in at least one of C-containing cohesive ends is methylated) are arranged on a substrate. In particular, when the contact of the DNA fragment mixture with the anti-methylated cytosine antibody in the antibody-contact step is carried out under conditions in which the monovalent binding is dissociated and the divalent binding is maintained in an antigen-antibody reaction, one or more nucleic acids capable of hybridizing with DNA fragments containing methylated cytosine in both C-containing cohesive ends at both ends are arranged on a substrate. When a combination of the anti-methylated cytosine antibody and the anti-cytosine antibody is used, or when DNA fragments obtained by performing the antibody-contact step under ordinary conditions followed by the elution under conditions in which the monovalent binding is dissociated and the divalent binding is maintained in an antigen-antibody reaction are identified, one or more nucleic acids capable of hybridizing with DNA fragments containing methylated cytosine in either C-containing cohesive end at both ends are arranged on a substrate.

In a DNA array produced by the methylated cytosine-type production method of preparing the DNA fragment mixture using the nucleic acid for single-stranded rendering, one or more nucleic acids capable of hybridizing with DNA fragments containing methylated cytosine in one or more single-stranded regions (i.e., DNA fragments in which at least one cytosine in one or more single-stranded regions is methylated) are arranged on a substrate. In particular, when the contact of the DNA fragment mixture with the anti-methylated cytosine antibody in the antibody-contact step is carried out under conditions in which the monovalent binding is dissociated and the divalent binding is maintained in an antigen-antibody reaction, one or more nucleic acids capable of hybridizing with DNA fragments containing two or more methylated cytosine in the single-stranded region(s) are arranged on a substrate. In this connection, when the nucleic acid for single-stranded rendering is used to prepare the DNA fragment mixture, it is preferable to convert both ends of the DNA fragments into blunt ends, to exclude influence of methylated cytosine or cytosine exposed at both ends.

In the methylated cytosine-type DNA array of the present invention, one or more nucleic acids capable of hybridizing with DNA fragments containing cytosine in a specific region (i.e., DNA fragments in which at least one cytosine in a specific region is not methylated), which may be used in the cytosine-type DNA array described below, may be arranged on a substrate. In the DNA array of the present invention, when one or more nucleic acids capable of hybridizing with DNA fragments containing methylated cytosine in a specific region, and one or more nucleic acids capable of hybridizing with DNA fragments containing cytosine in a specific region are arranged on a single substrate, methylation in plural methylation sites different in an extent of methylation can be comprehensively analyzed at a time.

### (2) Cytosine-type production method and cytosine-type DNA array

In the preparation step of the cytosine-type production method of the present invention, an appropriate DNA material is used to prepare a DNA fragment mixture in which one or more methylated cytosines or one or more cytosines to be methylated are exposed in each DNA fragment.
As the DNA material used in the preparation step, the DNA material previously mentioned in the preparation step of the methylated cytosine-type production method may be used.

In this connection, when genomic DNA in a cell is used, it is preferable to use a cell having a low methylation ratio, because DNA fragments to be arranged on a DNA array are selected on the basis of the presence or absence of cytosine in the cytosine-type production method. When the cell having a low methylation ratio is used, the DNA fragment mixture obtained in the preparation step may contain a high content of DNA fragments in which at least one cytosine is exposed.
As the cell having a low methylation ratio, there may be mentioned, for example, normal cells, ES cells, or tissue stem cells. Only one kind of cell may be used alone, or plural kinds of cells may be used as a combination thereof.

In the preparation step of the cytosine-type production method, as a method of preparing, from a DNA material, the mixture of DNA fragments in which methylated cytosine or cytosine to be methylated is exposed, the preparation methods previously mentioned in the preparation step of the methylated cytosine-type production method may be used. The descriptions with respect to the preparation methods which may be used in the preparation step of the methylated cytosine-type production method, except for the descriptions regarding the methylation-insensitive restriction enzyme capable of generating a C-containing cohesive end, may be applied to the preparation step of the cytosine-type production method.

When the methylation-insensitive restriction enzyme capable of generating a C-containing cohesive end is used in the preparation step of the cytosine-type production method, the genomic DNA can be fragmented with the restriction enzyme to generate cohesive ends and, simultaneously, methylated cytosine or cytosine to be methylated can be exposed at the cohesive ends.

Since the C (cytosine) in a 5'-CG-3' sequence is methylated in higher animals, it is preferable to use a methylation-insensitive restriction enzyme which generates the CG-containing cohesive end. In the cytosine-type production method, DNA fragments to be arranged on a DNA array are selected on the basis of the presence or absence of cytosine. Therefore, if cytosine which is not methylated is contained in the C-containing cohesive end, DNA fragments cannot be selected, because all DNA fragments contain cytosine. As such a restriction enzyme, which is not preferable, there may be mentioned, for example, BsaWI, BsoBI, or XmaI listed in Table 1. As a preferable enzyme which may be used in higher animals, there may be mentioned, for example, BssSI, MspI, or TaqI listed in Table 1.

Since the C (cytosine) in a 5'-CNG-3' sequence is methylated in plants, it is preferable to use a methylation-insensitive restriction enzyme which generates the CNG-containing cohesive end. As such a restriction enzyme, for example, restriction enzymes listed in Table 2 may be used. In BsaWI or XmaI listed in Table 2, there are two underlined "C"s to be methylated, as shown in Table 2. When BsaWI or XmaI is used and both "C"s in DNA fragments are methylated, the DNA fragments cannot react with an anti-cytosine antibody.

In the antibody-contact step of the cytosine-type production method, the DNA fragment mixture obtained in the preparation step is brought into contact with an anti-cytosine antibody, to separate a group consisting of DNA fragments which form an immunocomplex with the antibody (i.e., complex-forming DNA fragment group) from a group consisting of DNA fragments which do not react with the antibody (i.e., unreacted DNA fragment group), or to separate a group consisting of DNA fragments showing a high affinity for the antibody (i.e., high affinity DNA fragment group) from a group consisting of DNA fragments showing a low affinity for the antibody (i.e., low affinity DNA fragment group).

The anti-cytosine antibody may be prepared by using cytosine as an antigen in accordance with a conventional method, or a commercially available antibody may be used. For example, cytidine is bound to keyhole limpet hemocyanin to prepare an immunogen, and the immunogen is administered to a mouse to obtain the antibody of interest

The method of bringing the DNA fragment mixture into contact with the anti-cytosine antibody is not particularly limited, so long as the complex-forming DNA fragment group may be separated from the unreacted DNA fragment group. The complex-forming DNA fragment group may be separated from the unreacted DNA fragment group, for example, by bringing the DNA fragment mixture into contact with the anti-cytosine antibody carried on an appropriate carrier. Alternatively, after the DNA fragment mixture is brought into contact with the anti-cytosine antibody, the complex-forming DNA fragment group is purified from the whole in accordance with a conventional method to separate the complex-forming DNA fragment group from the unreacted DNA fragment group. In the former method using an appropriate carrier, the complex-forming DNA fragment group may be separated from the unreacted DNA fragment group, for example, by an affinity chromatography using the anti-cytosine antibody, or an antigen-antibody reaction using the anti-cytosine antibody carried on particles separable by centrifugation or magnetic particles. In the latter two-step method, the complex-forming DNA fragment group may be purified, for example, by using a column or particles (such as magnetic particles) carrying protein A or protein G thereon.

Among the mixture of DNA fragments in which methylated cytosine or cytosine to be methylated is exposed prepared in the preparation step, DNA fragments in which cytosine is exposed form an immunocomplex together with the anti-cytosine antibody, and are separated as the complex-forming DNA fragment group. DNA fragments in which cytosine is not exposed do not react with the anti-cytosine antibody, and are separated as the unreacted DNA fragment group.

When the DNA fragment mixture is prepared using the methylation-insensitive restriction enzyme capable of generating a C-containing cohesive end in the preparation step, the DNA fragments have C-containing cohesive ends at both ends. When the complex-forming DNA fragment group is separated from the unreacted DNA fragment group by contact with the anti-cytosine antibody, the DNA fragments contained in the complex-forming DNA fragment group include a DNA fragment in which both methylation sites located at both ends are not methylated (i.e., cytosine exists at both C-containing cohesive ends) and a DNA fragment in which only either methylation site at both ends is not methylated (i.e., cytosine exists at only either of two C-containing cohesive ends).

In this case (i.e., when the DNA fragment mixture is prepared using the methylation-insensitive restriction enzyme capable of generating a C-containing cohesive end), the contact of the DNA fragment mixture with the anti-cytosine antibody may be carried out under specific conditions to separate a complex-forming DNA fragment group consisting of only DNA fragments in which both methylation sites located at both ends are not methylated (i.e., cytosine exists at both C-containing cohesive ends) from an unreacted DNA fragment group.
With respect to the specific conditions, the descriptions, previously mentioned in the antibody-contact step of the methylated cytosine-type production method, with respect to the conditions in which the monovalent binding is dissociated and the divalent binding is maintained in an antigen-antibody reaction may be applied to the specific conditions used in the cytosine-type production method.

In the antibody-contact step, one or more DNA fragment groups different in the state of methylation of cytosine contained in the C-containing cohesive ends at both ends thereof may be obtained, by determining conditions of an antigen-antibody reaction, or selecting one or more antibodies or a combination thereof.
For example, a DNA fragment group consisting of DNA fragments in which at least one methylation site (i.e., cytosine) at both ends is not methylated (i.e., cytosine exists in at least one of two C-containing cohesive ends) may be obtained as the complex-forming DNA fragment group by carrying out an antigen-antibody reaction using the anti-cytosine antibody under ordinary conditions.
For example, a DNA fragment group consisting of DNA fragments in which both methylation sites (i.e., cytosine) at both ends are not methylated (i.e., cytosine exists at both C-containing cohesive ends) may be obtained as the complex-forming DNA fragment group by carrying out an antigen-antibody reaction using the anti-cytosine antibody under conditions in which the monovalent binding is dissociated and the divalent binding is maintained.
For example, a DNA fragment group consisting of DNA fragments in which either methylation site (i.e., cytosine) at both ends is not methylated (i.e., cytosine exists at either of two C-containing cohesive ends) may be obtained as the complex-forming DNA fragment group by carrying out an antigen-antibody reaction using a combination of the anti-cytosine antibody and the anti-methylated cytosine antibody under conditions in which the monovalent binding is dissociated and the divalent binding is maintained.

The identification step and the arrangement step of the cytosine-type production method may be carried out in a manner similar to that previously described in the identification step and the arrangement step of the methylated cytosine-type production method. The descriptions regarding the identification step and the arrangement step of the methylated cytosine-type production method may be applied to the identification step and the arrangement step of the cytosine-type production method.

In a DNA array produced by the cytosine-type production method of the present invention (i.e., the cytosine-type DNA array of the present invention), one or more nucleic acids capable of hybridizing with DNA fragments (among the DNA fragments contained in the DNA fragment mixture prepared in the preparation step) containing cytosine in a specific region (i.e., DNA fragments in which at least one cytosine in a specific region is not methylated) are arranged on a substrate. For example, when the methylation-insensitive restriction enzyme capable of generating a C-containing cohesive end is used, the specific region is the C-containing cohesive ends at both ends. When the nucleic acid for single-stranded rendering is used, the specific region is a target region of the nucleic acid for single-stranded rendering. In a DNA fragment containing a stem and loop structure, the specific region is a loop region.

More particularly, in a DNA array produced by the cytosine-type production method of preparing the DNA fragment mixture using the methylation-insensitive restriction enzyme capable of generating a C-containing cohesive end, one or more nucleic acids capable of hybridizing with DNA fragments containing cytosine in at least one of C-containing cohesive ends at both ends (i.e., DNA fragments in which at least one cytosine in at least one of C-containing cohesive ends is not methylated) are arranged on a substrate. In particular, when the contact of the DNA fragment mixture with the anti-cytosine antibody in the antibody-contact step is carried out under conditions in which the monovalent binding is dissociated and the divalent binding is maintained in an antigen-antibody reaction, one or more nucleic acids capable of hybridizing with DNA fragments containing cytosine in both C-containing cohesive ends at both ends are arranged on a substrate. When a combination of the anti-cytosine antibody and the anti-methylated cytosine antibody is used, or when DNA fragments obtained by performing the antibody-contact step under ordinary conditions followed by the elution under conditions in which the monovalent binding is dissociated and the divalent binding is maintained in an antigen-antibody reaction are identified, one or more nucleic acids capable of hybridizing with DNA fragments containing cytosine in either C-containing cohesive end at both ends are arranged on a substrate.

In a DNA array produced by the cytosine-type production method of preparing the DNA fragment mixture using the nucleic acid for single-stranded rendering, one or more nucleic acids capable of hybridizing with DNA fragments containing cytosine in one or more single-stranded regions (i.e., DNA fragments in which at least one cytosine in one or more single-stranded regions is not methylated) are arranged on a substrate. In particular, when the contact of the DNA fragment mixture with the anti-cytosine antibody in the antibody-contact step is carried out under conditions in which the monovalent binding is dissociated and the divalent binding is maintained in an antigen-antibody reaction, one or more nucleic acids capable of hybridizing with DNA fragments containing two or more cytosine in the single-stranded region(s) are arranged on a substrate. In this connection, when the nucleic acid for single-stranded rendering is used to prepare the DNA fragment mixture, it is preferable to convert both ends of the DNA fragments into blunt ends, to exclude influence of methylated cytosine or cytosine exposed at both ends.

In the cytosine-type DNA array of the present invention, one or more nucleic acids capable of hybridizing with DNA fragments containing methylated cytosine in a specific region (i.e., DNA fragments in which at least one cytosine in a specific region is methylated), which may be used in the methylated cytosine-type DNA array previously described, may be arranged on a substrate. In the DNA array of the present invention, when one or more nucleic acids capable of hybridizing with DNA fragments containing methylated cytosine in a specific region, and one or more nucleic acids capable of hybridizing with DNA fragments containing cytosine in a specific region are arranged on a single substrate, methylation in plural methylation sites different in an extent of methylation can be comprehensively analyzed at a time.

### (3) MONIC method

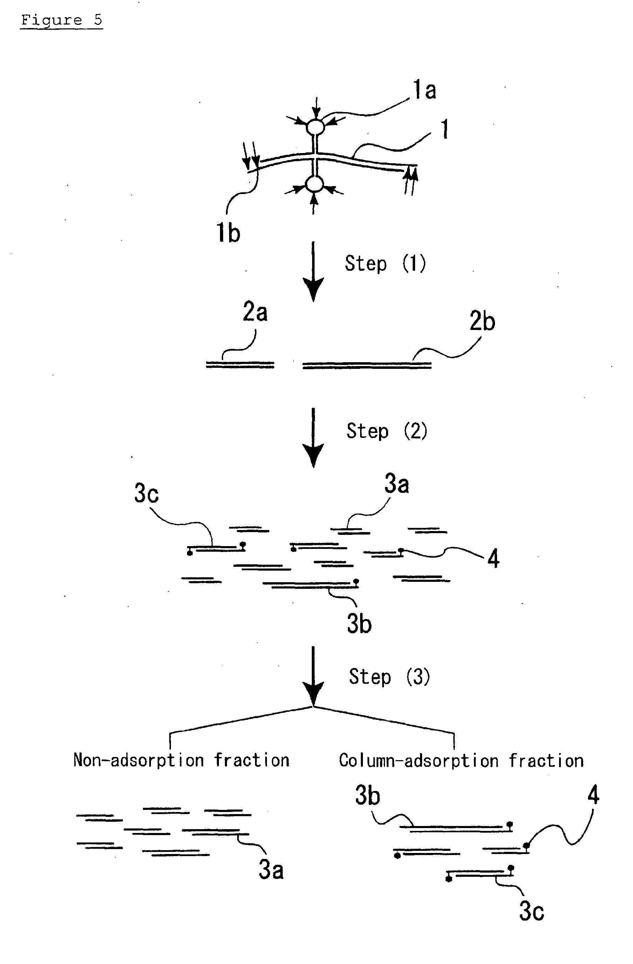

The MONIC method, an embodiment of the method of the present invention for producing a DNA array, will be illustrated with reference to Figure 5. In this connection, the following descriptions mainly refer to an embodiment using the anti-methylated cytosine antibody (i.e., methylated cytosine-type production method), but the MONIC method can be carried out using the anti-cytosine antibody.

In the MONIC method of the present invention, genomic DNA (or fragments thereof) 1 as a starting material may be treated with a nuclease capable of digesting a single-stranded DNA, before digesting with a restriction enzyme capable of generating a cohesive end (Step 1). In general, genomic DNA (or fragments thereof) 1 prepared from a biological sample contains one or more single-stranded structures (for example, a stem and loop structure 1a or a cohesive end 1b), which are naturally-occurring structures or artificial structures generated during procedures. When the procedure of step 1 is carried out, a mixture of DNA fragments 2a, 2b not having single-stranded structures but having blunt ends at both ends may be obtained. As the nuclease capable of digesting a single-stranded DNA, there may be mentioned, for example, Mung Bean nuclease or S1 nuclease.

The resulting mixture of blunt-ended DNA fragments may be digested with a restriction enzyme capable of generating a cohesive end containing methylated cytosine or cytosine to obtain a mixture of DNA fragments 3a, 3b, 3c in which methylated cytosine 4 or cytosine is exposed at cohesive ends (Step 2). The DNA fragment mixture contains DNA fragments 3a in which methylated cytosine does not exist at both cohesive ends, DNA fragments 3b in which methylated cytosine 4 exists at either cohesive end, and DNA fragments 3c in which methylated cytosine 4 exists at both cohesive ends.

The mixture of DNA fragments 3a, 3b, 3c is brought into contact with an anti-methylated cytosine antibody to separate a group consisting of DNA fragments which form an immunocomplex from an unreacted DNA fragment group (Step 3). For example, the mixture is passed through a column carrying the anti-methylated cytosine antibody to separate a fraction adsorbed by the column containing DNA fragments 3b, 3c from a non-adsorption fraction containing DNA fragments 3a. A combination of DNA fragments may be changed by selecting appropriate conditions of the column. For example, a column-adsorption fraction containing fragments 3c may be separated from a non-adsorption fraction containing fragments 3a, 3b.

Each obtained fraction may be used to analyze DNA fragments in accordance with a conventional method, and a DNA array may be produced on the basis of the results.

According to the MONIC method of the present invention, since genomic DNA is treated with the nuclease capable of digesting a single-stranded DNA, even when genomic DNA having stem and loop structures is used as a starting material, DNA fragments can be separated on the basis of the state of methylation at cohesive ends (or single-stranded structure) thereof, without influence of methylation sites which may be located in the stem and loop structures.

### (4) MONIC-Loop Trap method

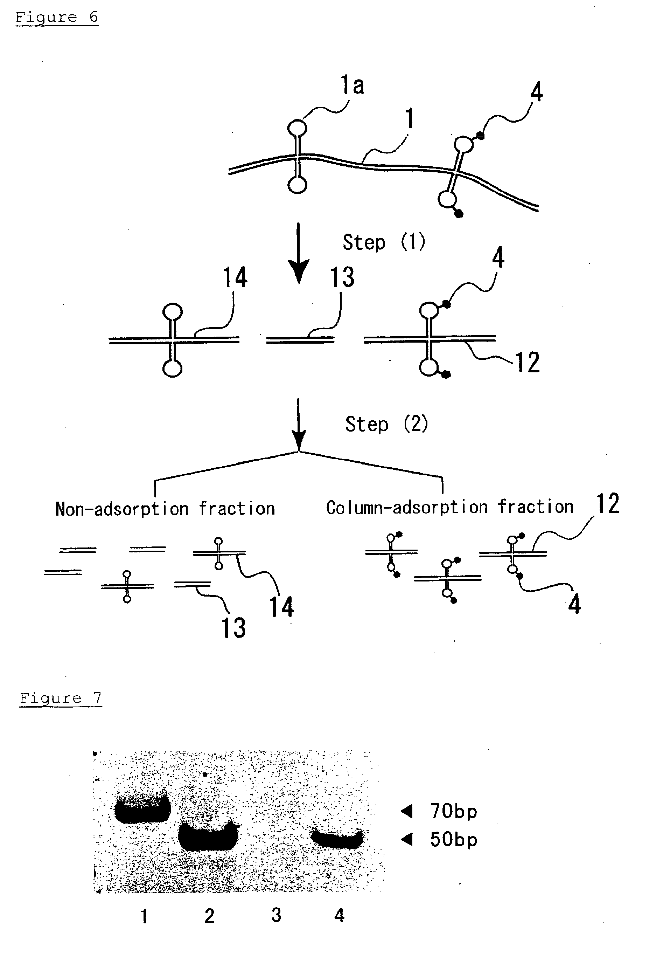

The MONIC-Loop Trap method, an embodiment of the method of the present invention for producing a DNA array, will be illustrated with reference to Figure 6. In this connection, the following descriptions mainly refer to an embodiment using the anti-methylated cytosine antibody (i.e., methylated cytosine-type production method), but the MONIC-Loop Trap method can be carried out using the anti-cytosine antibody.

In the MONIC-Loop Trap method of the present invention, genomic DNA (or fragments thereof) 1 as a starting material is treated with a restriction enzyme capable of generating a blunt end (Step 1). When the procedure of Step 1 is carried out, a mixture of DNA fragments 12, 13, 14 having blunt ends at both ends may be obtained. In the DNA fragments, the stem and loop structures 1a are maintained. The DNA fraction mixture contains DNA fragments having one or more stem and loop structures and DNA fragments 13 without a stem and loop structure. The DNA fragments having stem and loop structure(s) contain DNA fragments 12 in which methylated cytosine 4 is exposed in the loop region, and DNA fragments 14 in which methylated cytosine does not exist in the loop region.

The resulting DNA fragment mixture is brought into contact with an anti-methylated cytosine antibody to separate a group consisting of DNA fragments which form an immunocomplex from an unreacted DNA fragment group (Step 2). For example, the mixture is passed through a column carrying the anti-methylated cytosine antibody to separate a fraction adsorbed by the column containing DNA fragments 12 from a non-adsorption fraction containing DNA fragments 13, 14.
Each obtained fraction may be used to analyze DNA fragments in according to a conventional method, and a DNA array may be produced on the basis of the results.

### [2] Method of analyzing methylation according to the present invention

The method of the present invention for analyzing methylation comprises the preparation step, the antibody-contacting step, and the analysis step. In the method of the present invention for analyzing methylation, the DNA array of the present invention, or DNA arrays other than that of the present invention may be used.
On the basis of differences in specificities of antibodies used in the antibody-contact step, the method of the present invention for analyzing methylation includes a method of analyzing DNA fragments in which at least one methylated cytosine is exposed (hereinafter referred to as methylated cytosine-type analysis method), and a method of analyzing DNA fragments in which at least one cytosine is exposed (hereinafter referred to as cytosine-type analysis method).

In the methylated cytosine-type analysis method, included in the method of the present invention for analyzing methylation, at least an anti-methylated cytosine antibody (i.e., an antibody which specifically reacts with methylated cytosine, but does not specifically react with cytosine) is used as the antibody used in the antibody-contact step. It is preferable to use the methylated cytosine-type DNA array of the present invention as the DNA array used in the analysis step.

In the cytosine-type analysis method, included in the method of the present invention for analyzing methylation, at least an anti-cytosine antibody (i.e., an antibody which specifically reacts with cytosine, but does not specifically react with methylated cytosine) is used as the antibody used in the antibody-contact step. It is preferable to use the cytosine-type DNA array of the present invention as the DNA array used in the analysis step.
In this connection, the anti-methylated cytosine antibody and anti-cytosine antibody may be used as a combination thereof in the methylated cytosine-type or cytosine-type analysis method.
Hereinafter, the methylated cytosine-type analysis method will be explained, and then, the cytosine-type analysis method will be explained.

### (1) Methylated cytosine-type analysis method

In the preparation step of the methylated cytosine-type analysis method of the present invention, a DNA to be analyzed is used to prepare a DNA fragment mixture in which one or more methylated cytosines or one or more cytosines to be methylated are exposed in each DNA fragment.
The DNA to be analyzed in the methylated cytosine-type analysis method of the present invention is not particularly limited, so long as it is a DNA which may contain one or more methylated cytosines or one or more cytosines to be methylated. As the DNA to be analyzed, there may be mentioned, for example, genomic DNA in a cell (for example, an animal cell or a plant cell), or a mixture of free DNA fragments contained in a biological sample or a sample derived therefrom (for example, blood, plasma, serum, urine, lymph, a spinal fluid, saliva, a ascites fluid, an amniotic fluid, mucus, milk, bile, gastric juices, or an artificial dialysis fluid after dialysis).

As a method of preparing the DNA fragment mixture, the preparation methods previously mentioned in the preparation step of the methylated cytosine-type production method may be used. The descriptions with respect to the preparation methods which may be used in the preparation step of the methylated cytosine-type production method may be applied to the preparation step of the methylated cytosine-type analysis method.

In the antibody-contact step of the methylated cytosine-type analysis method, the DNA fragment mixture obtained in the preparation step is brought into contact with an anti-methylated cytosine antibody, to separate a group consisting of DNA fragments which form an immunocomplex with the antibody (i.e., complex-forming DNA fragment group) from a group consisting of DNA fragments which do not react with the antibody (i.e., unreacted DNA fragment group), or to separate a group consisting of DNA fragments showing a high affinity for the antibody (i.e., high affinity DNA fragment group) from a group consisting of DNA fragments showing a low affinity for the antibody (i.e., low affinity DNA fragment group).
The antibody-contact step of the methylated cytosine-type analysis method may be carried out in a manner similar to that previously described in the antibody-contact step of the methylated cytosine-type production method. The descriptions regarding the antibody-contact step of the methylated cytosine-type production method may be applied to the antibody-contact step of the methylated cytosine-type analysis method.

In the analysis step of the methylated cytosine-type analysis method of the present invention, DNA fragments contained in the complex-forming DNA fragment group and/or DNA fragments contained in the unreacted DNA fragment group obtained in the antibody-contact step are analyzed using a DNA array, which may be appropriately selected in accordance with an intended purpose of analysis.
As previously described in the antibody-contact step of the methylated cytosine-type production method or the cytosine-type production method, various DNA fragment groups, for example;
a DNA fragment group consisting of DNA fragments in which at least one methylation site (i.e., cytosine) at both ends is methylated (i.e., methylated cytosine exists in at least one of two C-containing cohesive ends); a DNA fragment group consisting of DNA fragments in which both methylation sites (i.e., cytosine) at both ends are methylated (i.e., methylated cytosine exists at both C-containing cohesive ends);
a DNA fragment group consisting of DNA fragments in which either methylation site (i.e., cytosine) at both ends is methylated (i.e., methylated cytosine exists at either of two C-containing cohesive ends);
a DNA fragment group consisting of DNA fragments in which at least one methylation site (i.e., cytosine) at both ends is not methylated (i.e., cytosine exists in at least one of two C-containing cohesive ends);
a DNA fragment group consisting of DNA fragments in which both methylation sites (i.e., cytosine) at both ends are not methylated (i.e., cytosine exists at both C-containing cohesive ends); or
a DNA fragment group consisting of DNA fragments in which either methylation site (i.e., cytosine) at both ends is not methylated (i.e., cytosine exists at either of two C-containing cohesive ends) may be obtained. In the present invention, one or more DNA fragment groups may be selected in accordance with an intended purpose of analysis.

As the DNA array used in the methylated cytosine-type analysis method of the present invention, there may be mentioned, for example, the methylated cytosine-type DNA array of the present invention, the cytosine-type DNA array of the present invention, or known DNA arrays. As known DNA arrays, there may be mentioned, for example, a DNA array in which nucleic acids capable of hybridizing with DNA fragments obtained by digesting genomic DNA with a methylation-insensitive restriction enzyme (for example, XmaI) capable of generating a cohesive end (for example, the CG-containing cohesive end) containing methylated cytosine or cytosine to be methylated are arranged in a substrate.

The DNA fragments contained in the complex-forming DNA fragment group may be analyzed, for example, while maintaining the form as the immunocomplex, or after separating the DNA fragments from the anti-methylated cytosine antibody, so long as the analysis is not affected. In general, the anti-methylated cytosine antibody is preferably used as the antibody immobilized on an appropriate carrier.

The analysis of the DNA fragments using the DNA array may be carried out in accordance with a conventional method. For example, the DNA fragments as a sample to be assayed are previously labeled with an appropriate label (such as a fluorescent substance or a radioactive substance), and hybridized with each nucleic acid arranged on the DNA array. Each signal derived from each labeled DNA fragment bound to each nucleic acid on the DNA array is analyzed (i.e., measured or detected) to comprehensively confirm the presence or absence of each DNA fragment capable of hybridizing with each nucleic acid. Alternately, the DNA fragments contained in the complex-forming DNA fragment group and the DNA fragments contained in the unreacted DNA fragment group may be independently labeled with different labels to subject them to the DNA array.

When the methylated cytosine-type DNA array of the present invention is used in the methylated cytosine-type analysis method of the present invention, for example, the DNA fragment mixture may be brought into contact with the anti-methylated cytosine antibody in the antibody-contact step to separate DNA fragments in which methylated cytosine is exposed (i.e., complex-forming DNA fragment group) from DNA fragments in which methylated cytosine is not exposed (i.e., unreacted DNA fragment group), and only the DNA fragments in which methylated cytosine is exposed may be used for the analysis using the DNA array. In this case, if a methylation ratio in genomic DNA of a cell to be analyzed is high, increased positive spots (i.e., decreased negative spots) on the DNA array are observed, because the DNA mixture contains a high content of DNA fragments in which methylated cytosine is exposed. If the methylation ratio is low, decreased positive spots (i.e., increased negative spots) on the DNA array are observed, because the DNA mixture contains a low content of DNA fragments in which methylated cytosine is exposed.
As above, on the basis of trends of positive spots and/or negative spots (i.e., numbers thereof) in each cell to be analyzed, the methylation ratio in genomic DNA of the cell can be evaluated. Further, on the basis of profiles of positive spots and/or negative spots, the state of the cell (for example, malignancy of a cancer cell, differentiation of a cell, morbidity of diseases) can be evaluated.

When a DNA array in which nucleic acids capable of hybridizing with DNA fragments obtained by digesting genomic DNA with the methylation-insensitive restriction enzyme (for example, XmaI) capable of generating a cohesive end containing methylated cytosine or cytosine to be methylated (for example, CG-containing cohesive end) are arranged on a substrate is used in the methylated cytosine-type analysis method of the present invention, for example, the DNA fragment mixture may be brought into contact with the anti-methylated cytosine antibody in the antibody-contact step to separate DNA fragments in which methylated cytosine is exposed (i.e., complex-forming DNA fragment group) from DNA fragments in which methylated cytosine is not exposed (i.e., unreacted DNA fragment group). Either group may be used for the analysis using the DNA array, or both groups independently labeled with different labels may be used for the analysis using the DNA array, to evaluate the state of methylation. If an amount of DNA fragments to be subjected to a DNA array is insufficient, a sufficient amount of DNA fragments may be obtained by previously performing a DNA amplification, such as a PCR, to carry out the analysis using a DNA array.
When the antibody-contact step is carried out under ordinary conditions, and an elution is carried out under conditions in which the monovalent binding is dissociated and the divalent binding is maintained in an antigen-antibody reaction, the complex-forming DNA fragment group consists of DNA fragments in which both methylation sites at both ends are methylated, and the eluted unreacted DNA fragment group consists of DNA fragments in which either methylation site at both ends is methylated. These DNA fragment groups (either or both) may be subjected to the DNA array to evaluate the state of methylation in detail.

According to the analysis method of the present invention, it is possible to analyze the state of methylation of each cell, and the kind of each cell can be mapped.
For example, almost all DNA fragments obtained by digesting genomic DNA with XmaI are considered to be a DNA in which methylation does not vary regardless of the kind of each cell. In particular, it is considered that parasite genes (not gene regions) should be always inactivated. It is preferable to notice a DNA fragment in which a DNA fragment derived from a cancer cell is trapped by a column, but a DNA fragment derived from a normal cell is not trapped, or a DNA fragment in which a DNA fragment derived from a normal cell is trapped by a column, but a DNA fragment derived from a cancer cell is not trapped.

### (2) Cytosine-type analysis method

In the preparation step of the cytosine-type analysis method of the present invention, a DNA to be analyzed is used to prepare a DNA fragment mixture in which one or more methylated cytosines or one or more cytosines to be methylated are exposed in each DNA fragment.
The DNA to be analyzed in the cytosine-type analysis method of the present invention is not particularly limited, so long as it is a DNA which may contain one or more methylated cytosines or one or more cytosines to be methylated. As the DNA to be analyzed, there may be mentioned, for example, genomic DNA in a cell (for example, an animal cell or a plant cell), or a mixture of free DNA fragments contained in a biological sample or a sample derived therefrom (for example, blood, plasma, serum, urine, lymph, a spinal fluid, saliva, a ascites fluid, an amniotic fluid, mucus, milk, bile, gastric juices, or an artificial dialysis fluid after dialysis).

As a method of preparing the DNA fragment mixture, the preparation methods previously mentioned in the preparation step of the methylated cytosine-type production method may be used. The descriptions with respect to the preparation methods which may be used in the preparation step of the methylated cytosine-type production method may be applied to the preparation step of the cytosine-type analysis method.

In the antibody-contact step of the cytosine-type analysis method, the DNA fragment mixture obtained in the preparation step is brought into contact with an anti-cytosine antibody, to separate a group consisting of DNA fragments which form an immunocomplex with the antibody (i.e., complex-forming DNA fragment group) from a group consisting of DNA fragments which do not react with the antibody (i.e., unreacted DNA fragment group), or to separate a group consisting of DNA fragments showing a high affinity for the antibody (i.e., high affinity DNA fragment group) from a group consisting of DNA fragments showing a low affinity for the antibody (i.e., low affinity DNA fragment group).
The antibody-contact step of the cytosine-type analysis method may be carried out in a manner similar to that previously described in the antibody-contact step of the cytosine-type production method. The descriptions regarding the antibody-contact step of the cytosine-type production method may be applied to the antibody-contact step of the cytosine-type analysis method.

In the analysis step of the cytosine-type analysis method of the present invention, DNA fragments contained in the complex-forming DNA fragment group and/or DNA fragments contained in the unreacted DNA fragment group obtained in the antibody-contact step are analyzed using a DNA array, which may be appropriately selected in accordance with an intended purpose of analysis.
The analysis step of the cytosine-type analysis method may be carried out in a manner similar to that previously described in the analysis step of the methylated cytosine-type analysis method. The descriptions regarding the analysis step of the methylated cytosine-type analysis method may be applied to the analysis step of the cytosine-type analysis method.

When the cytosine-type DNA array of the present invention is used in the cytosine-type analysis method of the present invention, for example, the DNA fragment mixture may be brought into contact with the anti-cytosine antibody in the antibody-contact step to separate DNA fragments in which cytosine is exposed (i.e., complex-forming DNA fragment group) from DNA fragments in which cytosine is not exposed (i.e., unreacted DNA fragment group), and only the DNA fragments in which cytosine is exposed may be used for the analysis using the DNA array. In this case, if a methylation ratio in genomic DNA of a cell to be analyzed is high, decreased positive spots (i.e., increased negative spots) on the DNA array are observed, because the DNA mixture contains a low content of DNA fragments in which cytosine is exposed. If the methylation ratio is low, increased positive spots (i.e., decreased negative spots) on the DNA array are observed, because the DNA mixture contains a high content of DNA fragments in which cytosine is exposed.
As above, on the basis of trends of positive spots and/or negative spots (i.e., numbers thereof) in each cell to be analyzed, the methylation ratio in genomic DNA of the cell can be evaluated. Further, on the basis of profiles of positive spots and/or negative spots, the state of the cell (for example, malignancy of a cancer cell, differentiation of a cell, morbidity of diseases) can be evaluated.

When a DNA array in which nucleic acids capable of hybridizing with DNA fragments obtained by digesting genomic DNA with the methylation-insensitive restriction enzyme (for example, XmaI) capable of generating a cohesive end containing methylated cytosine or cytosine to be methylated (for example, CG-containing cohesive end) are arranged on a substrate is used in the cytosine-type analysis method of the present invention, for example, the DNA fragment mixture may be brought into contact with the anti-cytosine antibody in the antibody-contact step to separate DNA fragments in which cytosine is exposed (i.e., complex-forming DNA fragment group) from DNA fragments in which cytosine is not exposed (i.e., unreacted DNA fragment group). Either group may be used for the analysis using the DNA array, or both groups independently labeled with different labels may be used for the analysis using the DNA array, to evaluate the state of methylation. If an amount of DNA fragments to be subjected to a DNA array is insufficient, a sufficient amount of DNA fragments may be obtained by previously performing a DNA amplification, such as a PCR, to carry out the analysis using a DNA array.
When the antibody-contact step is carried out under ordinary conditions, and an elution is carried out under conditions in which the monovalent binding is dissociated and the divalent binding is maintained in an antigen-antibody reaction, the complex-forming DNA fragment group consists of DNA fragments in which both methylation sites at both ends are not methylated, and the eluted unreacted DNA fragment group consists of DNA fragments in which either methylation site at both ends is not methylated. These DNA fragment groups (either or both) may be subjected to the DNA array to evaluate the state of methylation in detail.

The main features of the methylated cytosine-type analysis method (an embodiment using the methylated cytosine-type array) and the cytosine-type analysis method (an embodiment using the cytosine-type array), which are included in the method of the present invention for analyzing methylation, are shown in Table 3.

**Table 3**

| Analysis method | | Methylated cytosine-type | Cytosine-type |
|---|---|---|---|
| Antibody used | | Anti-methylated cytosine antibody | Anti-cytosine antibody |
| DNA array used | | Methylated cytosine-type | Cytosine-type |
| Trends of spots | High methylation | Positive spots | Negative spots |
| | Low methylation | Negative spots | Positive spots |

### [3] Method of purifying DNA of the present invention

In the method of the present invention for purifying a DNA, a double-stranded DNA fragment having cohesive ends is obtained using an antibody specific to a base contained in the cohesive ends. The method of the present invention for purifying a DNA may be used, for example, in the antibody-contact step of the production method of the present invention or the method of the present invention for analyzing methylation.

As a known method of purifying a DNA fragment having cohesive ends (for example, DNA fragments obtained by digesting a DNA with a restriction enzyme), for example, a method of separating DNA fragments in a gel by electrophoresis and eluting the DNA fragment of interest from the gel is commonly used. However, since the electrophoresis is an essential step in this method, the DNA fragment of interest cannot be purified rapidly.

Various antibodies specific to each base contained in a DNA, and a method of isolating a single-stranded DNA using the same are known, but a method of isolating DNA fragments obtained by digesting a DNA with a restriction enzyme using such an antibody has never been attempted. To the knowledge of the inventor, the reasons are considered to be as follows. In a double-stranded DNA, since each base is located inside of main chains, it is considered that the binding of an anti-base antibody to the base as the antigen would be inhibited. Further, since the base length of a cohesive end generated by a restriction enzyme is generally 2 to 4 bases, it is considered that the binding of an anti-base antibody to the base located in a cohesive end is difficult due to steric hindrance or the like.

The present inventor examined various embodiments of the antibody-contact step in the production method of the present invention and, as a result, found that a DNA fragment having a cohesive end can be isolated by using an antibody specific to a base contained in the cohesive end. The result in which the antigen-antibody reaction at such a short cohesive end can be carried out without influence of steric hindrance or the like is unexpected.

A double-stranded DNA fragment which may be used in the method of purifying a DNA according to the present invention is not particularly limited, so long as it has at least one cohesive end. As the double-stranded DNA fragment, there may be mentioned, for example, DNA fragments obtained by digesting a DNA with a restriction enzyme capable of generating a cohesive end, DNA fragments obtained by digesting a DNA with an exonuclease, or a double-stranded DNA fragment obtained by hybridizing two kinds of single-stranded DNAs. The base length of the cohesive end is not particularly limited, but may be, for example, 10 bases or less, preferably a base length (generally 5 bases or less, preferably 4 bases or less, more preferably 3 bases or less, most preferably 2 bases or less) of a cohesive end generated by a digestion with a restriction enzyme.

As the base contained in a cohesive end, there may be mentioned, for example, general bases contained in a naturally-occurring DNA (for example, cytosine, methylated cytosine, adenine, guanine, or thymine), bases chemically modified or chemically converted by oxidation (for example, 8-oxoguanine, generated by an oxidation at the 8-position of the purine ring in guanine), or bases crosslinked by ultraviolet rays (for example, thymidine dimer). As the antibody specific to a base contained in a cohesive end, there may be mentioned, for example, an anti-cytosine antibody, an anti-methylated cytosine antibody, an anti-adenine antibody, an anti-guanine antibody, an anti-thymine antibody, an anti-8-oxoguanine antibody, or an anti-thymidine dimer antibody. The antibody may be selected in accordance with a nucleotide sequence of a cohesive end of a DNA fragment to be purified.
The antibody may be a monoclonal antibody or a polyclonal antibody. The term "antibody" includes not only an antibody in a narrow sense (i.e., an immunoglobulin molecule per se), but also a fragment of an antibody, such as Fab, Fab', F(ab')₂, or Fv.

The contact of the DNA fragment to be purified with the anti-base antibody may be carried out in a manner similar to that of a general antigen-antibody reaction. When the contact is carried out under conditions in which the monovalent binding is dissociated and the divalent binding is maintained in an antigen-antibody reaction, a DNA fragment having the same cohesive end at both ends may be separated from the other DNA fragments. For example, when a DNA is digested with a combination of two or more kinds of restriction enzymes, a mixture of DNA fragments having various cohesive ends generated by the restriction enzymes is obtained. An antigen-antibody reaction may be carried out using the DNA fragment mixture under conditions in which the monovalent binding is dissociated and the divalent binding is maintained in an antigen-antibody reaction, to separate DNA fragments having the same cohesive end at both ends from other DNA fragments (for example, DNA fragments having different cohesive ends at both ends).

According to the method of purifying a DNA according to the present invention, only DNA fragments containing a specific base at cohesive ends can be purified, on the basis of the presence or absence of the specific base, without performing gel electrophoresis.
For example, a plasmid containing a cloned DNA fragment is treated with two kinds of restriction enzymes A and B to excise and purify the DNA fragment. The purified DNA fragment having a cohesive end generated by the restriction enzyme A and another cohesive end generated by the restriction enzyme B is further divided with the third restriction enzyme C into two subfragments. Either subfragment may be obtained on the basis of the presence or absence of a specific base contained in a nucleotide sequence generated by the restriction enzyme A or B, without performing gel electrophoresis.
Further, the method of purifying a DNA according to the present invention is used to amplify only one or more DNA fragments (including a case of a kind of DNA fragment and a case of a mixture of plural kinds of DNA fragments) containing a specific base in a cohesive end. Hereinafter the method of the present invention for amplifying a DNA using the method of purifying a DNA according to the present invention will be illustrated.

In the method of amplifying a DNA according to the present invention, for example, a DNA sample is digested with a restriction enzyme capable of generating a cohesive end, and only one or more DNA fragments of interest are purified using an antibody. Since the purified DNA fragment(s) have cohesive ends, the DNA fragments may be religated with a DNA ligase to generate a DNA having a high molecular weight. In this case, the purified DNA fragment(s) may be a kind of DNA fragment, or a mixture of plural kinds of DNA fragments. After the resulting high molecular weight DNA is amplified using an appropriate DNA amplification system, the amplified DNA may be re-digested with the restriction enzyme used in the first digestion to selectively amplify only DNA fragment(s) previously purified by using the antibody.

As the DNA amplification system, there may be mentioned, for example, an amplification system using GenomiPhi (for example, GenomiPhi DNA Amplification Kit, Cat. #25-6600-01; Amersham). According to the amplification system, a DNA having a base length of 50 kbp or more can be efficiently amplified. For example, several nanograms of DNA can be amplified to several micrograms of DNA. Since random primers are used, all DNA fragments previously purified by using the antibody can be uniformly amplified. The DNA amplification system is not particularly limited, so long as all DNA fragments obtained may be amplified at a time. The same result may be obtained, for example, by ligating a linker adaptor to the DNA fragments purified by using the antibody and amplifying the ligated DNA fragments by a PCR method using oligo-DNA primers specific to the linker adaptor.

According to the method of amplifying a DNA according to the present invention, a high S/N ratio can be obtained, in comparison with a DNA amplification using DNAs containing various genes as a template, because the DNA(s) of interest are concentrated before the amplification. This indicates that the content of the DNA(s) of interest in the amplified DNAs can be easily judged.
Until now, an amplified DNA product in which the state of methylation of cytosine in a template DNA is maintained has not been obtained. However, in the present invention, the DNA(s) of interest are concentrated and purified by the antibody using, as an index, methylation of cytosine in cohesive ends (i.e., single-stranded DNA regions) at both ends generated by a digestion of a restriction enzyme, before the DNA amplification, and thus, only methylated DNA(s) can be amplified.
As above, even if a large amount of DNA fragments in which cytosine in single-stranded cohesive ends is not methylated are contained in a DNA sample to be assayed, DNA fragments in which cytosine in single-stranded cohesive ends is methylated can be efficiently amplified.

For example, in a patient suffering from a cancer, a very small amount of cancer-specifically methylated DNA fragments flow from a cancer tissue into blood. In a healthy person not suffering from a cancer, several nanograms/mL of DNA fragments derived from normal cells flow in blood. To analyze whether or not a subject is suffering from a cancer, it is necessary to detect abnormal methylated DNA fragments derived from a cancer in the DNA fragments derived from normal cells.
According to the present invention, cancer-specifically methylated DNA fragments can be concentrated using an antibody. In the concentrated and purified DNA fraction, DNA fragments having abnormal methylation caused by canceration are concentrated.

### EXAMPLES

The present invention now will be further illustrated by, but is by no means limited to, the following Examples.

### Example 1: Production of methylated cytosine-type DNA array

### (1) Separation of XmaI-digested DNA fraction

Genomic DNA was extracted from a human normal fibroblast TIG-1 [population doubling level (PDL) = 29 to 30] in accordance with a conventional method, and 0.5 µg of genomic DNA was digested with a restriction enzyme XmaI (10 U) for 2 hours. The digested DNA fragments were diluted with 1 mL of 10 mmol/L phosphate buffer (PB)/50 mmol/L NaCl/0.05% Tween 20 (pH 7.15) to dilute a reducing agent contained in a buffer for a restriction enzyme. To the solution, 10 µg of an anti-5-methylcytcsine mouse monoclonal antibody [Japanese Unexamined Patent Publication No. 2004-347508 (JP 2004-347508 A1); or Program and Abstracts in 25th Annual Meeting of the Molecular Biology Society of Japan, published on November 25, 2002, 2P-0111, p.717] was added, and the whole allowed to stand at room temperature for 5 minutes. After the reaction, the whole was passed through a protein A column [CIM monolithic column Protein A HLD; BIA Separations d.o.o.(Slovenia); http://www.monoliths.com/]. The column was washed twice with 10 mmol/L PB/0.15 mol/L NaCl/0.05% Tween 20 (2.5 mL) to remove non-adsorbed DNA fragments from the column. Next, 10 mmol/L PB/0.4 mmol/L NaCl/0.05% Tween 20 (4 mL) was passed through the column to elute DNA fragments, which dissociated under these conditions, from the column, and the fraction was kept as the first DNA fraction. After the column was washed with the same buffer (5 mL), all remaining DNA fragments were eluted with 2 mL of 0.3 mol/L sodium acetate (pH 4.5), and the fraction was kept as the second DNA fraction.

A total amount of each DNA fraction was measured, and the first and second DNA fractions were 16.7 ng and 7.2 ng, respectively. In this connection, 10 ug of antibody was used in this example. When an amount of antibody to be added is increased, an amount of DNA which binds to the column is increased and, as a result, an increased yield of DNA may be obtained. Further, when a length of the column is lengthened, an increased yield of DNA may be obtained.

### (2) Amplification and cloning of DNA fragments and production of DNA array

To each fraction obtained in Example 1(1), 1/10 volume of 3 mol/L sodium acetate and an 2.5-fold amount of ethanol are added. The mixture is allowed to stand at -20°C for an hour, and centrifuged to collect DNAs as a precipitate. The precipitated DNAs are ligated in a conventional method to generate DNAs having a high molecular weight. The DNAs are amplified using a commercially available amplification system (GenomiPhi DNA Amplification Kit, Cat. #25-6600-01; Amersham). The amplified DNAs are redigested with XmaI, and each DNA fragment contained therein is cloned and sequenced. Oligonucleotides to be arranged on a DNA array are designed and chemically synthesized on the basis of the determined nucleotide sequences, taking Tm into consideration. The synthesized oligonucleotides are arranged on a substrate in a conventional method to produce the DNA array of the present invention.

### Example 2: Isolation of DNA fragments by MONIC method

### (1) Preparation of DNA fragment mixture

Genomic DNA was purified from a human B-cell derived tumor cell line (Burkitt lymphoma Raji cell; JCRB9012, JCRB cell bank) in a conventional method, and dissolved in 30 µL of 10 mmol/L Tris-HCl (pH 8.0)/100 mmol/L NaCl/25 mmol/L EDTA/0.5% SDS. To the DNA solution, a proteinase K solution in 50% glycerin solution (20 mg/mL) was added so that a final concentration of proteinase K became 0.4 mg/mL. The mixture was incubated at 55°C for 2 hours, and an phenol/chloroform extraction and an ethanol precipitation were carried out in accordance with conventional methods to obtain DNAs. After 30 µg of the DNAs was dissolved in a buffer [30 mmol/L sodium acetate (pH 5.0), 1000 mmol/L NaCl, 1 mmol/L zinc acetate, and 10% glycerol], Mung Bean nuclease (Takara Bio) was added at a concentration of 1 U/µg DNA to the solution, and the mixture was incubated at 37°C for 30 minutes. By this treatment, single-stranded regions of genomic DNA are removed, and all ends are completely converted into blunt ends.

The reaction mixture was diluted with 300 µL of a TE buffer [a 10 mmol/L Tris-HCl buffer (pH 8.0) and 0.5 mmol/L EDTA], and filtered using a filter for adsorbing proteins (MicropureEZ; MILLIPORE) for a short time to remove the Mung Bean nuclease. The obtained filtrate was concentrated to approximately 10 µL using an ultrafilter (MicroconYM-5; MILLIPORE), and digested with a restriction enzyme BsaJI (New England Biolab; 2 Unit/1 µg DNA) at 60°C for 1.5 hours to obtain DNA fragments.

### (2) Isolation of DNA fragments

The obtained DNA fragment mixture may be subjected to an affinity column carrying an anti-5-methylcytosine antibody to obtain a DNA fraction of interest.

### Example 3: Isolation of DNA fragments by MONIC-Loop Trap method

To a 1.5-mL tube, 500 µL of a suspension containing 4×10⁷ magnetic beads (Dynabeads; Dynal biotech) was added, and washed with PBS [a 10 mmol/L phosphate buffer (pH 7.5) and 0.15 mol/L NaCl], several times. To the tube, 500 µL of PBS containing 4 µg of the same anti-5-methylcytosine mouse monoclonal antibody (clone 1-5GB4A5) as that used in Example 1 and 0.1% bovine serum albumin (BSA) was added, and the reaction was carried out using a rotator at room temperature for 30 minutes. The magnetic beads were collected using a magnet, to remove a supernatant.

The genomic DNA prepared in Example 2 was digested with a restriction enzyme AluI, and 2 µg of the obtained DNA fragments was dissolved in PBS to adjust to 500 µL. The solution was added to the collected magnetic beads, and the mixture was allowed to stand at room temperature for 1 hour. After a supernatant was removed, 500 µL of PBS was added to the magnetic beads, and the magnetic beads were washed for ten minutes while reversing gently by a rotator. The washing treatment was repeated three times. To the magnetic beads, 500 µL of an elution buffer [a 10 mmol/L phosphate buffer (pH 6.5) and 0.15 mol/L NaCl] was added, and suspended gently. The magnetic beads were collected using a magnet, and a supernatant was collected as an adsorption fraction. For comparison, the genomic DNA previously treated with Mung Bean nuclease was digested with the restriction enzyme AluI (capable of generating a blunt end), and the obtained DNA fragments were brought into contact with the magnetic beads. However, no DNA fragments captured by the magnetic beads were observed.

### E_{X}ample 4: Isolation of DNA fragments by MONIC method using magnetic beads

The DNA fragment mixture (i.e., DNA fragments obtained by the treatment with Mung Bean nuclease followed by the treatment with the restriction enzyme BsaJI) obtained in Example 1(2) was used to carry out the isolation procedure using the magnetic beads described in Example 3. The obtained adsorption fraction, and the starting DNA fragment mixture before adsorption were used as templates to carry out a PCR. As primer sets for the PCR, a primer set A of oligonucleotides having the nucleotide sequences of SEQ ID NO: 1 (a sense primer) and SEQ ID NO: 2 (a reverse primer), and a primer set B of oligonucleotides having the nucleotide sequences of SEQ ID NO: 3 (a sense primer) and SEQ ID NO: 4 (a reverse primer) were used. The amplified products were analyzed by an 8% acrylamide gel electrophoresis.

The primer sets A and B can be used to amplify a 70-bp DNA fragment (-238th to -169th bases) and a 50-bp DNA fragment (+11th to +60th bases) in a promoter region of a human E-cadherin gene, respectively. In this connection, the starting base of exon 1 is represented as "+1". The 70-bp DNA fragment amplified by the primer set A does not contain a CpG sequence at both ends, and thus, does not contain methylated cytosine at both ends. In contrast, the 50-bp DNA fragment amplified by the primer set B contains CpG sequences at both ends, and thus, contains methylated cytosine at both ends. With respect to the methylation of the CpG sequences, it was reported that the promoter region of the human E-cadherin gene in the cell line used in this Example was highly methylated. Further, the present inventor confirmed the methylation of the CpG sequences by another method.

The result of electrophoresis is shown in Figure 7. In Figure 7, lanes 1 and 2 show the results when the starting DNA fragment mixture before adsorption was used as a template, and lanes 3 and 4 show the results when the adsorption fraction was used as a template. Further, lanes 1 and 3 show the results when the primer set A was used (a number of the CpG sequences at both ends: 0), and lanes 2 and 4 show the results when the primer set B was used (a number of the CpG sequences at both ends: 2).

As shown in Figure 7, the starting DNA fragment mixture before adsorption contained a DNA fragment (70 bp) not containing methylated cytosine at both ends and a DNA fragment (50 bp) containing methylated cytosine at both ends. In contrast, the adsorption fraction contained the DNA fragment (50 bp) containing methylated cytosine at both ends, but did not contain the DNA fragment (70 bp) not containing methylated cytosine at both ends. It was confirmed that the DNA fragment (50 bp) containing methylated cytosine at both ends could be separated from the DNA fragment (70 bp) not containing methylated cytosine at both ends by the method of the present invention.

## Claims

1. A method of producing a DNA array, **characterized by** comprising the steps of:
(1) preparing a mixture of DNA fragments in which a modified base or a base is exposed, wherein the mixture of DNA fragments is
(a) a mixture of DNA fragments in which a modified base or a base is exposed at a cohesive end thereof, obtained by digesting genomic DNA with a restriction enzyme which can digest a DNA regardless of the presence or absence of a modification in a recognition site to generate a cohesive end containing a modified base or a base, wherein the genomic DNA is pretreated with a nuclease capable of digesting a single-stranded DNA, before digesting the genomic DNA with the restriction enzyme, or
(b) a mixture of single-stranded DNA fragments or partially single-stranded DNA fragments in which a modified base or a base is exposed in the single-stranded region, obtained by fragmenting genomic DNA and rendering the fragmented genomic DNAs fully or partially single-stranded, wherein the genomic DNA or the fragmented genomic DNAs are pretreated with a nuclease capable of digesting a single-stranded DNA, before rendering the fragmented genomic DNAs fully or partially single-stranded.
(2) bringing the mixture of DNA fragments obtained in step (1) into contact with an antibody specific to either the modified base or to the corresponding base, and separating the mixture into a group consisting of DNA fragments which form an immunocomplex with the antibody and another group consisting of DNA fragments which do not react with the antibody, or a group consisting of DNA fragments showing a high affinity for the antibody and another group consisting of DNA fragments showing a low affinity for the antibody,
(3) identifying all or part of DNA fragments contained in each of the DNA fragment groups, and
(4) arranging one or more nucleic acids capable of hybridizing with any one of the identified DNA fragments on a substrate.

2. The method according to claim 1, wherein, when the mixture of DNA fragments is brought into contact with the antibody in the step (2), at least one antigen-antibody reaction is performed under conditions in which a monovalent binding is dissociated and a divalent binding is maintained, to separate the mixture into a group consisting of DNA fragments capable of binding to the antibody by a divalent binding, as the group consisting of DNA fragments which form an immunocomplex with the antibody, and another group consisting of DNA fragments capable of binding to the antibody by a monovalent binding, as the group consisting of DNA fragments which do not react with the antibody.

3. The method according to claim 1, wherein, when the mixture of DNA fragments is brought into contact with the antibody in the step (2), at least one antigen-antibody reaction is performed under conditions in which a group consisting of DNA fragments showing a high affinity can be separated from another group consisting of DNA fragments showing a low affinity on the basis of the difference between a monovalent binding and a divalent binding, to separate the mixture into a group consisting of DNA fragments capable of binding to the antibody by a divalent binding, as the group consisting of DNA fragments showing a high affinity, and another group consisting of DNA fragments capable of binding to the antibody by a monovalent binding, as the group consisting of DNA fragments showing a low affinity.

4. A method of analyzing a modification in a DNA to be assayed, **characterized by** comprising the steps of:
(1) preparing a mixture of DNA fragments in which a modified base or a base is exposed, from the DNA to be assayed, wherein the mixture of DNA fragments is
(a) a mixture of DNA fragments in which a modified base or a base is exposed at a cohesive end thereof, obtained by digesting genomic DNA with a restriction enzyme which can digest a DNA regardless of the presence or absence of a modification in a recognition site to generate a cohesive end containing a modified base or a base, wherein the genomic DNA is pretreated with a nuclease capable of digesting a single-stranded DNA, before digesting the genomic DNA with the restriction enzyme, or
(b) a mixture of single-stranded DNA fragments or partially single-stranded DNA fragments in which a modified base or a base is exposed in the single-stranded region, obtained by fragmenting genomic DNA and rendering the fragmented genomic DNAs fully or partially single-stranded, wherein the genomic DNA or the fragmented genomic DNAs are pretreated with a nuclease capable of digesting a single-stranded DNA, before rendering the fragmented genomic DNAs fully or partially single-stranded.
(2) bringing the mixture of DNA fragments obtained in the step (1) into contact with an antibody specific to either the modified base or to the corresponding base, and separating the mixture into a group consisting of DNA fragments which form an immunocomplex with the antibody and another group consisting of DNA fragments which do not react with the antibody, or a group consisting of DNA fragments showing a high affinity for the antibody and another group consisting of DNA fragments showing a low affinity for the antibody, and
(3) analyzing all or part of DNA fragments contained in each of the DNA fragment groups with a DNA array obtainable by the method according to any one of claims 1 to 3.

## Patentansprüche

1. Verfahren zur Herstellung eines DNA-Arrays, **dadurch gekennzeichnet, dass** es die Stufen:
1) Herstellen eines Gemischs aus DNA-Fragmenten, bei welchen eine modifizierte Base oder eine Base freigelegt wird, wobei das Gemisch aus DNA-Fragmenten
a) ein Gemisch aus DNA-Fragmenten, bei welchen eine modifizierte Base oder eine Base an einem kohäsiven Ende davon wirdund welche durch Verdauen genomischer DNA mit einem Restriktionsenzym erhalten worden sind, das eine DNA unabhängig von dem Vorhandensein oder Fehlen einer Modifikation an einer Erkennungsstelle verdauen kann, um ein eine modifizierte Base oder eine Base enthaltendes kohäsives Ende zu erzeugen, wobei die genomische DNA vor ihrer Verdauung mit dem Restriktionsenzym mit einer Nuclease vorbehandelt wird, die in der Lage ist, eine einsträngige DNA zu verdauen, oder
b) ein Gemisch aus einsträngigen DNA-Fragmenten oder teilweise einsträngigen DNA-Fragmenten ist, bei welchen eine modifizierte Base oder eine Base in der einsträngigen Region freigelegt wird und welche **dadurch** erhalten worden sind, dass genomische DNA fragmentiert wurde und die fragmentierten genomischen DNAs vollständig oder teilweise einsträngig gemacht worden sind, wobei die genomische DNA oder die fragmentierten genomischen DNAs mit einer Nuclease vorbehandelt werden, die in der Lage ist, eine einsträngige DNA zu verdauen, bevor die fragmentierten genomischen DNAs vollständig oder teilweise einsträngig gemacht werden,
2) In-Berührung-Bringen des in Stufe 1) erhaltenen Gemischs aus DNA-Fragmenten mit einem Antikörper, der entweder für die modifizierte Base oder die korrespondierende Base spezifisch ist, und Auftrennen des Gemischs in eine Gruppe, die aus DNA-Fragmenten, die mit dem Antikörper einen Immunkomplex bilden, besteht, und in eine andere Gruppe, die aus DNA-Fragmenten, die nicht mit dem Antikörper reagieren, besteht, oder eine Gruppe, die aus DNA-Fragmenten, die eine hohe Affinität zu dem Antikörper aufweisen, besteht, und eine andere Gruppe, die aus DNA-Fragmenten, die eine geringe Affinität zu dem Antikörper aufweisen, besteht,
3) Identifizieren aller DNA-Fragmente, die in jeder DNA-Fragment-Gruppe enthalten sind, oder eines Teils davon und
4) Anordnen einer oder mehrerer Nucleinsäuren, die in der Lage sind, mit einem beliebigen der identifizierten DNA-Fragmente zu hybridisieren, auf einem Substrat umfasst.

2. Verfahren nach Anspruch 1, wobei, wenn das Gemisch aus DNA-Fragmenten mit dem Antikörper in Stufe 2) in Berührung gebracht wird, mindestens eine Antigen-Antikörper-Reaktion unter Bedingungen stattfindet, bei welchen eine einfache Bindung aufgespalten wird und eine zweifache Bindung erhalten bleibt, um das Gemisch in eine Gruppe, die aus DNA-Fragmenten besteht, die in der Lage sind, durch eine zweifache Bindung an den Antikörper zu binden, als die Gruppe, die aus DNA-Fragmenten besteht, die mit dem Antikörper einen Immunkomplex bilden, und eine andere Gruppe, die aus DNA-Fragmenten besteht, die in der Lage sind, durch eine einfache Bindung an den Antikörper zu binden, als die Gruppe, die aus DNA-Fragmenten besteht, die nicht mit dem Antikörper reagieren, aufzutrennen.

3. Verfahren nach Anspruch 1, wobei, wenn das Gemisch aus DNA-Fragmenten mit dem Antikörper in Stufe 2) in Berührung gebracht wird, mindestens eine Antigen-Antikörper-Reaktion unter Bedingungen stattfindet, bei welchen eine Gruppe, die aus DNA-Fragmenten, die eine hohe Affinität aufweisen, besteht, von einer anderen Gruppe, die aus DNA-Fragmenten, die eine geringe Affinität aufweisen, besteht, auf der Grundlage des Unterschieds zwischen einer einfachen Bindung und einer zweifachen Bindung abgetrennt werden kann, um das Gemisch in eine Gruppe, die aus DNA-Fragmenten besteht, die in der Lage sind, durch eine zweifache Bindung an den Antikörper zu binden, als die Gruppe, die aus DNA-Fragmenten besteht, die eine hohe Affinität aufweisen, und eine andere Gruppe, die aus DNA-Fragmenten besteht, die in der Lage sind, durch eine einfache Bindung an den Antikörper zu binden, als die Gruppe, die aus DNA-Fragmenten besteht, die eine geringe Affinität aufweisen, aufzutrennen.

4. Verfahren zur Analyse einer Modifikation in einer zu untersuchenden DNA, **dadurch gekennzeichnet, dass** es die Stufen:
1) Herstellen eines Gemischs aus DNA-Fragmenten der zu untersuchenden DNA, bei welchen eine modifizierte Base oder eine Base freigelegt wird, wobei das Gemisch aus DNA-Fragmenten
a) ein Gemisch aus DNA-Fragmenten, bei welchen eine modifizierte Base oder eine Base an einem kohäsiven Ende davon freigelegt wird und welche durch Verdauen genomischer DNA mit einem Restriktionsenzym erhalten worden sind, das eine DNA unabhängig von dem Vorhandensein oder Fehlen einer Modifikation an einer Erkennungsstelle verdauen kann, um ein eine modifizierte Base oder eine Base enthaltendes kohäsives Ende zu erzeugen, wobei die genomische DNA vor ihrer Verdauung mit dem Restriktionsenzym mit einer Nuclease vorbehandelt wird, die in der Lage ist, eine einsträngige DNA zu verdauen, oder
b) ein Gemisch aus einsträngigen DNA-Fragmenten oder teilweise einsträngigen DNA-Fragmenten ist, bei welchen eine modifizierte Base oder eine Base in der einsträngigen Region freigelegt wird und welche **dadurch** erhalten worden sind, dass genomische DNA fragmentiert wurde und die fragmentierten genomischen DNAs vollständig oder teilweise einsträngig gemacht worden sind, wobei die genomische DNA oder die fragmentierten genomischen DNAs mit einer Nuclease vorbehandelt werden, die in der Lage ist, eine einsträngige DNA zu verdauen, bevor die fragmentierten genomischen DNAs vollständig oder teilweise einsträngig gemacht werden,
2) In-Berührung-Bringen des in Stufe 1) erhaltenen Gemischs aus DNA-Fragmenten mit einem Antikörper, der entweder für die modifizierte Base oder die korrespondierende Base spezifisch ist, und Auftrennen des Gemischs in eine Gruppe, die aus DNA-Fragmenten, die mit dem Antikörper einen Immunkomplex bilden, besteht, und in eine andere Gruppe, die aus DNA-Fragmenten, die nicht mit dem Antikörper reagieren, besteht, oder eine Gruppe, die aus DNA-Fragmenten, die eine hohe Affinität zu dem Antikörper aufweisen, besteht, und eine andere Gruppe, die aus DNA-Fragmenten, die eine geringe Affinität zu dem Antikörper aufweisen, besteht, und
3) Analysieren aller DNA-Fragmente, die in jeder DNA-Fragment-Gruppe enthalten sind, oder eines Teils davon mit einem DNA-Array, das durch das Verfahren nach einem der Ansprüche 1 bis 3 erhältlich ist,
umfasst.

## Revendications

1. Méthode de production d'un réseau d'ADN, **caractérisée en ce qu'**elle comprend les étapes de :
(1) préparation d'un mélange de fragments d'ADN dans lesquels une base modifiée ou une base est exposée, dans laquelle le mélange de fragments d'ADN est :
(a) un mélange de fragments d'ADN dans lesquels une base modifiée ou une base est exposée à une extrémité cohésive, obtenue par digestion d'ADN génomique avec une enzyme de restriction qui peut digérer un ADN indépendamment de la présence ou de l'absence d'une modification dans un site de reconnaissance pour générer une extrémité cohésive contenant une base modifiée ou une base, dans lequel l'ADN génomique est prétraité avec une nucléase capable de digérer un ADN simple-brin, avant la digestion de l'ADN génomique avec l'enzyme de restriction, ou
(b) un mélange de fragments d'ADN simple-brin ou de fragments d'ADN partiellement simple-brin dans lesquels une base modifiée ou une base est exposée dans une région simple-brin, obtenue par fragmentation de l'ADN génomique et rendant les ADN génomiques complètement ou partiellement simple-brin, dans lequel l'ADN génomique ou les ADN génomiques fragmentés sont prétraités avec une nucléase capable de digérer un ADN simple brin, avant de rendre les ADN génomiques fragmentés complètement ou partiellement simples-brins,
(2) mise en contact du mélange des fragments d'ADN obtenus dans l'étape (1) avec un anticorps spécifique soit vis-à-vis de la base modifiée soit vis à vis la base correspondante, et séparation du mélange en un groupe constitué des fragments d'ADN qui forment un immuno-complexe avec l'anticorps et un autre groupe constitué des fragments ne réagissant pas avec l'anticorps, ou un groupe constitué des fragments d'ADN montrant une forte affinité pour l'anticorps et un autre constitué des fragments d'ADN montrant une faible affinité pour l'anticorps,
(3) identification de tout ou partie des fragments d'ADN contenus dans chacun des groupes de fragments d'ADN, et
(4) arrangement d'un ou plusieurs acides nucléiques capables de s'hybrider avec l'un des fragments d'ADN identifié sur un substrat.

2. Méthode selon la revendication 1, dans laquelle, lorsque le mélange de fragments d'ADN est mis en contact avec l'anticorps dans l'étape (2), au moins une réaction antigène-anticorps est effectuée dans des conditions selon lesquelles une liaison monovalente est dissociée et une liaison divalente est maintenue, pour séparer le mélange en un groupe constitué de fragments d'ADN capables de liaison avec l'anticorps par une liaison divalente, tel un groupe constitué de fragments d'ADN formant un immuno-complexe avec l'anticorps, et un autre groupe constitué de fragments d'ADN capables de se lier à l'anticorps par une liaison monovalente, tel le groupe constitué de fragments d'ADN ne réagissant pas avec l'anticorps.

3. Méthode selon la revendication 1, dans laquelle, lorsque le mélange de fragments d'ADN est mis en contact l'anticorps dans l'étape (2), au moins une réaction antigène-anticorps est effectuée dans des conditions dans lesquelles un groupe constitué de fragments d'ADN montrant une grande affinité peut être séparé d'un autre groupe constitué de fragments d'ADN montrant une faible affinité sur la base de la différence entre liaison monovalente et liaison divalente, pour séparer le mélange en un groupe constitué de fragments d'ADN capables de se lier à l'anticorps par une liaison divalente, tel le groupe constitué de fragments montrant une haute affinité, et un autre groupe constitué de fragments d'ADN capables de se lier à l'anticorps par une liaison monovalente, comme le groupe constitué de fragments d'ADN montrant une faible affinité.

4. Méthode d'analyse d'une modification dans un ADN à tester, **caractérisée en ce qu'**elle comprend les étapes de :
(1) préparation d'un mélange de fragments d'ADN dans lesquels une base modifiée ou une base est exposée, de l'ADN à tester, dans laquelle le mélange de fragments d'ADN est :
(a) un mélange de fragments d'ADN dans lesquels une base modifiée ou une base est exposée à extrémité cohésive, obtenue par digestion d'ADN génomique avec une enzyme de restriction qui peut digérer un ADN indépendamment de la présence ou de l'absence d'une modification dans un site de reconnaissance pour générer une extrémité cohésive contenant une base modifiée ou une base, dans le quel l'ADN génomique est prétraité avec une nucléase capable de digérer un ADN simple-brin, avant la digestion de l'ADN génomique avec l'enzyme de restriction, ou
(b) un mélange de fragments d'ADN simple-brin ou de fragments d'ADN partiellement simple-brin dans lesquels une base modifiée ou une base est exposée dans une région simple-brin, obtenue par fragmentation de l'ADN génomique et rendant les ADN génomiques complètement ou partiellement simple-brin, dans lequel l'ADN génomique ou les ADN génomiques fragmentés sont prétraités avec une nucléase capable de digérer un ADN simple brin, avant de rendre les ADN génomiques fragmentés complètement ou partiellement simples-brins,
(2) mise en contact du mélange des fragments d'ADN obtenus dans l'étape (1) avec un anticorps spécifique soit vis-à-vis de la base modifiée soit vis à vie de la base correspondante, et séparation du mélange en un groupe constitué des fragments d'ADN qui forment un immuno-complexe avec l'anticorps et un autre groupe constitué des fragments ne réagissant pas avec l'anticorps, ou un groupe constitué des fragments d'ADN montrant une forte affinité pour l'anticorps et un autre constitué des fragments d'ADN montrant une faible affinité pour l'anticorps, et
(3) analyse de tout ou partie des fragments d'ADN contenu dans chaque groupe de fragments d'ADN avec un réseau d'ADN par la méthode décrite dans l'une quelconque des revendications 1 à 3.


### SEQUENCE LISTING

<110> JAPAN SCIENCE AND TECHNOLOGY AGENCY
<110> TOKYO METROPOLITAN FOUNDATION FOR RESEARCH ON AGING AND PROMOTION OF HUMAN WELFARE
<120> DNA array for analyzing DNA methylation, process for manufacturing the same, and method for analyzing DNA methylation
<130> TMI-724
<150> JP 2004-044759
   <151> 2004-02-20
<160> 4
<170> PatentIn version 3.1
<210> 1
   <211> 20
   <212> DNA
   <213> Homo sapiens
<400> 1
   attcgaaccc agtggaatca 20
<210> 2
   <211> 20
   <212> DNA
   <213> Homo sapiens
<400> 2
   ctagcctgga gttgctaggg 20
<210> 3
   <211> 20
   <212> DNA
   <213> Homo sapiens
<400> 3
   gaactgcaaa gcacctgtga 20
<210> 4
   <211> 19
   <212> DNA
   <213> Homo sapiens
<400> 4
   gggctggagt ctgaactga 19